# EUROPEAN PATENT APPLICATION

(11) **EP 4 431 652 A1**
(43) Date of publication of application: **18.09.2024**
(21) Application number: 22901761.1
(22) Date of filing: 30.11.2022
(51) Int. Cl.: D06F 58/12, D06F 58/26, D06F 73/02, D06F 19/00, A61L 2/07

(54) **CLOTHES TREATMENT APPARATUS**

(30) Priority: 30.11.2021 KR 20210168358
(71) Applicant: LG Electronics Inc., Yeongdeungpo-gu Seoul 07336 (KR)
(72) Inventor: CHONG, Chungook, Seoul 08592 (KR); SEO, Bumjune, Seoul 08592 (KR)
(74) Representative: Schott, Jakob Valentin
(86) International application number: PCT/KR2022/019181
(87) International publication number: WO 2023/101403

(57) **Abstract**

The present disclosure relates to a clothes treatment apparatus in which a rotary shaft for shaking clothes is disposed in the width direction of an inner case and a power transmission part for shaking the clothes while rotating in the axial direction according to the rotation of the rotary shaft is provided, wherein the rotary shaft is prevented from being exposed to the inside of the inner case.

## Description

### [Technical Field]

The present disclosure relates to a laundry treatment apparatus, and more particularly to a laundry treatment apparatus capable of removing foreign substances such as dust by applying physical force to clothes accommodated therein.

### [Background Art]

Generally, a laundry treatment apparatus may refer to a device for washing clothes, drying wet or washed clothes, and/or removing wrinkles from clothes at home or at a laundromat. The laundry treatment apparatus may conceptually include a washing machine for washing clothes, a dryer for drying wet or washed clothes, a washing/drying machine for performing both of washing and drying of clothes, a refresher for deodorizing and refreshing clothes, a steamer for removing wrinkles from clothes, and the like.

Recently, a laundry treatment apparatus has appeared that allows clothes to be kept pleasant and clean without washing the clothes with detergent by soaking the clothes in water. Such a conventional laundry treatment apparatus can shake off fine dust attached to clothes, deodorize clothes, dry clothes, and add fragrance to clothes. In addition, the laundry treatment apparatus can prevent occurrence of static electricity, can remove wrinkles from clothes using dehumidified air or steam, and can sterilize and disinfect clothes.

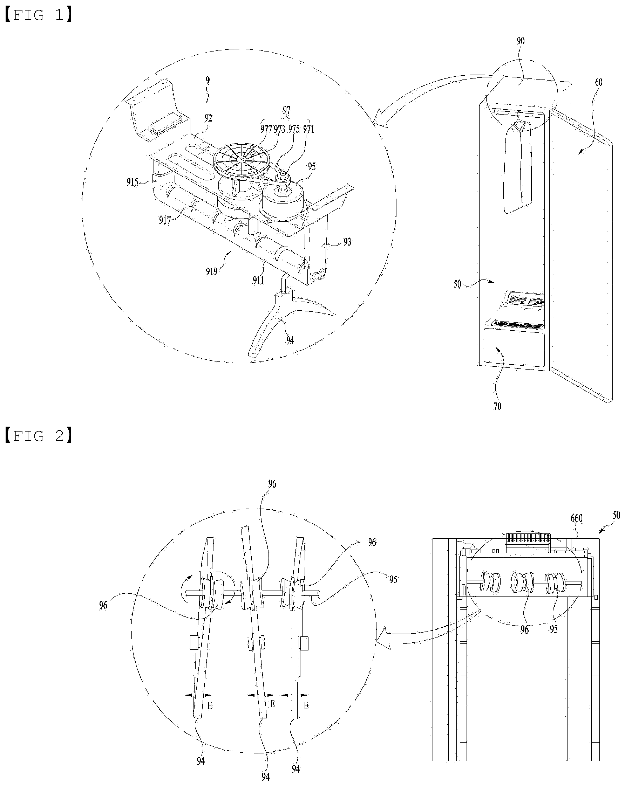

FIG. 1 is a diagram illustrating a conventional laundry treatment apparatus disclosed in Korean Patent Registration No. 10-1525568.

Referring to FIG. 1, a conventional laundry treatment apparatus may include a cabinet 50 forming an exterior appearance thereof and having an opening disposed at the front thereof, an inner case (T) provided inside the cabinet and having a space for accommodating clothes therein, a door 60 for opening or closing the opening, and a supply unit 70 provided below the door 60 to supply hot air or steam into the cabinet 50.

The conventional laundry treatment apparatus can refresh clothes hung on a clothes hanger 94 in the cabinet by supplying hot air or steam from the supply unit 70.

However, the conventional laundry treatment apparatus has difficulty in removing fine dust attached to clothes using only hot air or steam. Since hot air or steam is not evenly exposed to clothes, the conventional laundry treatment apparatus cannot guarantee refreshing performance.

Therefore, the conventional laundry treatment apparatus may further include a drive unit 90 capable of vibrating clothes by shaking the clothes hanger 94.

The drive unit 90 may include a frame 92 fixed to and supported by the cabinet, a motor 95 seated in the frame 92 to generate rotational power, and a transmission unit 97 configured to transmit rotational power of the motor 95, a switching unit 977 configured to convert the rotational power transmitted by the transmission unit 97 into a rectilinear reciprocating motion, a support bar 919 configured to shake due to movement of the conversion unit 977, and a support rib 93 fixed to the frame 92 fixing the support bar 919.

The transmission unit 97 may include a belt 975 and a pulley 973 coupled to a rotary shaft 971 of the motor. The switching unit 977 may be provided with a cam structure for converting rotational motion into reciprocating rectilinear motion.

The support bar 919 may include a support groove 917 in which the support bar 911 and the clothes hanger 94 are seated. When the motor 95 rotates, the support bar 919 may vibrate in the width direction of the cabinet due to movement of the switching unit 977.

As a result, the clothes hanger 94 may shake off fine dust from clothes by horizontally vibrating along the support bar 919, so that the area in which hot air or steam can come in contact with clothes hung on the clothes hanger 94 can increase in size.

However, the conventional laundry treatment apparatus has disadvantages in that, in order to overcome inertial force at a time point where the support bar 919 changes the movement direction thereof, strong vibration or noise unavoidably may occur due to characteristics that the support bar 919 rectilinearly reciprocates along the width direction of the cabinet.

For example, when the support bar 919 moves to the left of the cabinet and then changes to the right, or when the support bar 919 moves to the right and then changes to the left of the cabinet, strong vibration may occur.

That is, when the support bar 919 moves quickly to the left and then changes to the right, the speed instantly becomes zero '0' and then rapidly accelerates to the right again. In addition, when the support bar 919 moves quickly to the right and then changes to the left, the speed of the support bar 919 instantly becomes zero '0' and then rapidly accelerates to the left again.

Accordingly, strong vibration is generated while the speed and direction of the support bar 91 rapidly change, and this vibration is transmitted to the cabinet 50 to shake the cabinet 50 so that there occur problems that vibration and noise are generated to the outside of the cabinet 50.

In addition, such vibration not only weakens the durability of the drive unit 90, but also is transmitted to clothes such that clothes collide with each other or are shaken strongly, thereby causing damage to the clothes.

In order to address this issue, a laundry treatment apparatus that does not rapidly change the movement direction of clothes, such as Japanese Patent Registration No. 6355075, has been proposed.

FIG. 2 is a diagram illustrating a conventional laundry treatment apparatus to which a new driving method is applied.

Referring to FIG. 2, the laundry treatment apparatus may include a rotary shaft 95 configured to extend in the width direction of the inner case within the cabinet, and a plate 96 obliquely coupled to the rotary shaft 95.

The plate 96 may include an inclined bearing coupled to be inclined with respect to the axial direction of the rotary shaft 95.

Accordingly, the inclined bearing coupled to the inside of the plate 96 may process with respect to the rotary shaft 95, but the plate 96 may be movable to change only the axial direction without rotating together with the rotary shaft 95.

The axial direction of the plate 96 may be regularly changed as if the plate 96 has moved along a conical shape, and only the movement direction of the plate 96 may change but the movement speed of the plate 96 may not vary greatly. Accordingly, only the movement direction of the clothes hanger 94 mounted on the plate 96 changes, but the movement speed of the clothes hanger 94 may be uniform or may not be greatly changed.

Therefore, since strong inertial force is not generated in the clothes hanger 94, occurrence of strong vibration in the clothes hanger 94 can be prevented.

When the clothes hanger 94 is mounted on the plate 96, the clothes hanger 94 may shake as the axial direction of the plate 96 changes.

The axial direction of the plate 96 does not change abruptly by continuous rotation of the rotary shaft 95 but changes with a uniform acceleration. As a result, the clothes hanger 94 can also be shaken while moving at a uniform acceleration.

Specifically, the clothes hanger 94 may be continuously twisted with respect to the side surface of the cabinet 50 by a change in the axial direction of the plate 96 or may move back and forth (i.e., pendulum motion) with respect to the plate 96.

As a result, there are disadvantages in that the rotary shaft 95 is exposed to hot air or steam and corroded, and the user may unexpectedly touch the rotary shaft 95 so that the user could get burned by the overheated rotary shaft 95 or could be injured due to the moving rotary shaft 95.

In particular, if a child or the like hangs on the rotary shaft 95 for fun or heavy clothes are simultaneously hung on the rotary shaft 95, the rotary shaft 95 is bent by heavy weight so that the rotary shaft 95 cannot rotate by the motor and the ability to shake clothes is rapidly deteriorated.

On the other hand, in such a laundry treatment apparatus, the clothes hanger 94 may be directly mounted on the plate 96. Since the clothes hanger 94 does not have a separate support member, the clothes hanger cannot move integrally with the plate 96 due to its own inertial force.

In addition, the conventional laundry treatment apparatus shown in FIG. 1 has disadvantages in that the clothes hanger 94 is only mounted on the support bar without being fixed to or supported by the support bar such that the clothes hanger 94 cannot move integrally with the support bar 911.

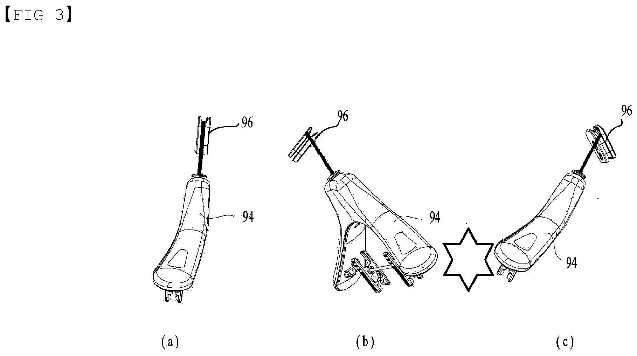

FIG. 3 is a diagram illustrating the problem that the clothes hanger and the drive unit cannot move integrally in the conventional laundry treatment apparatus.

Referring to FIG. 3(a), only a hook portion of the clothes hanger 94 can be mounted on the plate 96 without being separately supported by the plate 96.

Referring to FIGS. 3(b) and 3(c), even if the axial direction of the plate 96 is changed such that the plate 96 can shake the clothes hanger 94, the clothes hanger 94 has no choice but to move later than the plate 96 due to its own inertial force.

For example, when the plate 96 guides the clothes hanger 94 to the left, the clothes hanger 94 cannot move instantaneously, so that the clothes hanger 94 may be inclined to the right with respect to the plate 96.

In addition, when weights of clothes hung on the plurality of clothes hangers 94 are different from each other, since there is a difference in inertial force between the clothes hangers 94, the plurality of clothes hangers 94 can shake with different amplitudes in different directions.

Accordingly, the respective clothes hangers 94 may be disposed to be inclined by different angles with respect to the plate 96, so that the clothes hangers 94 may collide with each other or the clothes hung on the clothes hangers 94 may also collide with each other, thereby causing damage to the clothes.

The above-described problem may also occur when the clothes hangers 94 are mounted on the support bar 911 and are shaken horizontally.

Moreover, when the hook portion of each clothes hanger 94 is simply caught in a groove provided at the upper end of the plate 96, force of the plate 96 may not be transmitted to the clothes hanger 94 without change.

In other words, the hook portion of the clothes hanger 94 is not fixed to the plate 96 but is placed in the plate 96 such that the hook portion of the clothes hanger 94 can rotate with a certain degree of freedom. As a result, although the axial direction of the plate 96 is changed in all of the back-and-forth direction, the left-and-right direction, and the up-and-down direction, the clothes hanger 94 inevitably shakes only in the left-and-right direction due to its own weight and inertial force.

Therefore, even if the plate 96 moves in multiple directions, the clothes hanger 94 may move only in one direction, so that the conventional laundry treatment apparatus cannot guarantee the effect of shaking the entire surface of clothes in multiple directions.

### [Disclosure]

### [Technical Problem]

An object of the present disclosure is to provide a laundry treatment apparatus that is disposed in the width direction of the inner case that accommodates clothes and prevents a rotary shaft provided to agitate clothes from being bent.

Another object of the present disclosure is to provide a laundry treatment apparatus capable of supporting a load applied to the rotary shaft.

Another object of the present disclosure is to provide a laundry treatment apparatus that ensures continuous rotation of the rotary shaft and can stably agitate a plurality of clothes even when the plurality of clothes is placed on the rotary shaft.

Another object of the present disclosure is to provide a laundry treatment apparatus that can prevent deformation of the rotary shaft or damage to the rotary shaft even in abnormal situations where a load such as a child is suddenly applied to the rotary shaft.

### [Technical Solutions]

In order to solve the above-described issues, the laundry treatment apparatus according to the present disclosure may include a support unit for supporting the rotary shaft at a lower portion of the rotary shaft that transmits power for agitating the clothes.

The rotary shaft may be arranged in parallel in the width direction of the cabinet, and at least a portion of the support unit may be disposed lower than the rotary shaft to support the load of the rotary shaft.

As a result, the support unit can strengthen the durability of the rotary shaft by distributing the load applied to the rotary shaft.

The support unit may directly support the rotary shaft, or may indirectly support the rotary shaft by supporting a constituent component coupled to the rotary shaft.

Specifically, the support unit may include a support plate coupled to the cabinet or the inner case so that at least a portion of the support plate is disposed at a lower position than the rotary shaft.

The support plate may include: a support holder that supports at least one of the power transmission unit and the holder to distribute a load applied to the rotary shaft.

The support holder may include: a passage hole that passes through the support plate to allow any one of the power transmission unit and the holder to pass therethrough; and a seating surface provided on an outer circumferential surface of the passage hole to support a bottom surface of the power transmission unit or the holder.

The power transmission unit may include: a bearing unit having an outer circumferential surface that is obliquely coupled to the rotary shaft so that an extension direction of the outer circumferential surface rotates about the rotary shaft; and a rigid body coupled to the outer circumferential surface of the bearing unit to rotate independently of the bearing unit and configured to support the holder, wherein the seating surface is provided as a curved surface so that the seating surface supports the rigid body unit while allowing movement of the rigid body unit.

The seating surface may be recessed from the support plate to support the rigid body unit.

The width of the seating surface may be greater than a recess depth of the seating unit.

The width of the seating surface may be greater than a thickness of an outer circumferential surface of the rigid body unit.

The seating surface may be formed in a funnel shape.

The radius of curvature of the seating surface may be greater than or equal to a radius of curvature of a trajectory along which an outer circumferential surface of the rigid body unit moves.

The radius of curvature of the seating surface may be greater than or equal to a radius of curvature of an outer circumferential surface of the rigid body unit.

The support holder may include a reinforcement surface protruding from the support plate toward the rigid body unit, wherein the seating surface is provided inside the reinforcement unit.

The height at which the reinforcement surface may protrude from the support plate is greater than a depth at which the seating surface is recessed.

The seating surface may be provided to support the holder.

The holder may include: a coupling plate coupled to the rigid body unit and formed to extend into the inner case by penetrating the passage hole. The coupling plate may include: a coupling end coupled to the rigid body unit; an extension end extending from the coupling end unit and exposed to the inside of the inner case by penetrating the passage hole; and a holder support unit protruding to be larger than a diameter of the passage hole and seated on the seating surface.

The holder support may include: a holder body provided to have a larger thickness than the coupling end unit; and a body surface provided at a lower portion of the holder body and supported by the seating surface.

The seating surface may be provided to be larger than a region of the body surface, and a radius of curvature of the seating surface may be equal to or greater than a radius of curvature of the body surface.

The support unit may include a support pillar coupled to the support plate and supporting a lower portion of the rotary shaft.

The support pillar may include: a pillar body coupled to the support plate; and a pillar groove provided at an upper portion of the pillar body to receive and support the rotary shaft.

The support pillar may be implemented as a plurality of support pillars that is spaced apart from each other by a preset distance in a longitudinal direction of the rotary shaft.

The support plate may further include: a passage hole through which one of the power transmission unit and the holder pass and supporting the one of the power transmission unit and the holder, the plurality of support pillars is arranged to be spaced apart from the passage hole.

The support unit may further include a rotary coupling unit coupled to the support plate to rotatably support both ends of the rotary shaft.

In order to solve the above-described issues, a laundry treatment apparatus may include the support unit. The support unit may be provided to contact the rotary shaft at a lower position than the rotary shaft or to accommodate and support the rotary shaft.

The support unit may include: a support plate provided below the rotary shaft; and a support pillar provided on the support plate and supporting a lower portion of the rotary shaft.

The support pillar may be provided to support a region between both ends of the rotary shaft.

The support plate may include a rotary coupling unit configured to rotatably support both ends of the rotary shaft; and the support pillar may be disposed between the rotary coupling units.

The support plate may include a through-hole through which the holder passes; and the support pillar may be arranged to be spaced apart from the through-hole.

In order to solve the above-described issues, the laundry treatment apparatus may include a support unit. The support unit may be provided to support any one of the power transmission unit and the holder to distribute a load applied to the rotary shaft.

The support unit may include: a support plate fixed to the cabinet and disposed at a lower position than the rotary shaft; a passage hole formed to penetrate the support plate to allow any one of the power transmission unit and the holder to pass therethrough; and a support holder extending from an outer circumferential surface of the through-hole to support at least a portion of the power transmission unit and the holder disposed above the through hole.

### [Advantageous Effects]

As is apparent from the above description, the laundry treatment apparatus is disposed in the width direction of the inner case that accommodates clothes and prevents a rotary shaft provided to agitate clothes from being bent.

The laundry treatment apparatus according to the present disclosure can support a load applied to the rotary shaft.

The laundry treatment apparatus according to the present disclosure may ensure continuous rotation of the rotary shaft and may thus stably shake a plurality of clothes even when the plurality of clothes is placed on the rotary shaft.

The laundry treatment apparatus according to the present disclosure can prevent deformation of the rotary shaft or damage to the rotary shaft even in abnormal situations where a load such as a child is suddenly applied to the rotary shaft.

### [Description of Drawings]

FIG. 1 is a view illustrating a conventional laundry treatment apparatus disclosed in Korean Patent Registration No. 10-1525568.
FIG. 2 is a view illustrating a conventional laundry treatment apparatus to which a new driving method is applied.
FIG. 3 is a view illustrating a problem that the clothes hanger and the drive unit cannot move integrally in the conventional laundry treatment apparatus.
FIG. 4 is a view illustrating one example of the laundry treatment apparatus according to the present disclosure.
FIG. 5 is a view illustrating an example of a drive unit of the laundry treatment apparatus according to an embodiment of the present disclosure.
FIG. 6 is a view illustrating one example of a hanger support 430.
FIG. 7 is a view illustrating an example of a drive unit of the laundry treatment apparatus according to another embodiment of the present disclosure.
FIG. 8 is a view illustrating an example of a power transmission unit of the laundry treatment apparatus according to the present disclosure.
FIG. 9 is a view illustrating a process for driving the power transmission unit of the laundry treatment apparatus according to the present disclosure.
FIGS. 10 and 11 are views illustrating states of the clothes hangers moving by the power transmission unit of the laundry treatment apparatus according to the present disclosure.
FIG. 12 is a view illustrating a situation in which clothes hangers 800 do not move integrally with the holder 400.
FIG. 13 is a view illustrating one example of the power transmission unit and the holder.
FIG. 14 is a cross-sectional view illustrating a coupling structure between the power transmission unit and the holder shown in FIG. 13.
FIG. 15 is a view illustrating an example of a detailed structure of the power transmission unit and the holder shown in FIG. 13.
FIG. 16 is a view illustrating another example of the power transmission unit and the holder.
FIG. 17 is a cross-sectional view illustrating a coupling structure between the power transmission unit and the holder shown in FIG. 16.
FIG. 18 is a view illustrating an example of a detailed structure of the power transmission unit and the holder shown in FIG. 16.
FIG. 19 is a view illustrating an example of a support unit for supporting the power transmission unit or the rotary shaft.
FIG. 20 is a view illustrating an embodiment of a support holder 540.
FIG. 21 is a view illustrating a specific shape of the support holder 540.
FIG. 22 is a view illustrating another example of the support 500.
FIG. 23 is a view illustrating a detailed structure of a coupling plate 413 applied to the structure of FIG. 22.
FIG. 24 is a view illustrating an example of the coupling plate 413 performing a pendulum motion.
FIG. 25 is a view illustrating an example of the support 500 directly supporting the rotary shaft 210.
FIG. 26 is a view illustrating a sealing unit of the laundry treatment apparatus according to the present disclosure.
FIG. 27 is a view illustrating an example of the sealing unit.
FIG. 28 is a view illustrating operation methods of a first sealing unit 701 according to the present disclosure.
FIG. 29 is a view illustrating another example of the sealing unit 700 of the laundry treatment apparatus according to the present disclosure.
FIG. 30 is a view illustrating other examples of a second sealing unit 702 according to the present disclosure.

### [Best Mode]

Reference will now be made in detail to the embodiments of the present disclosure, examples of which are illustrated in the accompanying drawings. Wherever possible, the same reference numbers will be used throughout the drawings to refer to the same or similar parts. A singular expression may include a plural expression unless otherwise stated in the context. In the following description, a detailed description of related known configurations or functions incorporated herein will be omitted to avoid obscuring the subject matter. The accompanying drawings illustrate the exemplary embodiments of the present disclosure. The exemplary embodiments of the present disclosure are merely provided to describe the present disclosure in detail, and the technical range of the present disclosure is not limited by the exemplary embodiments.

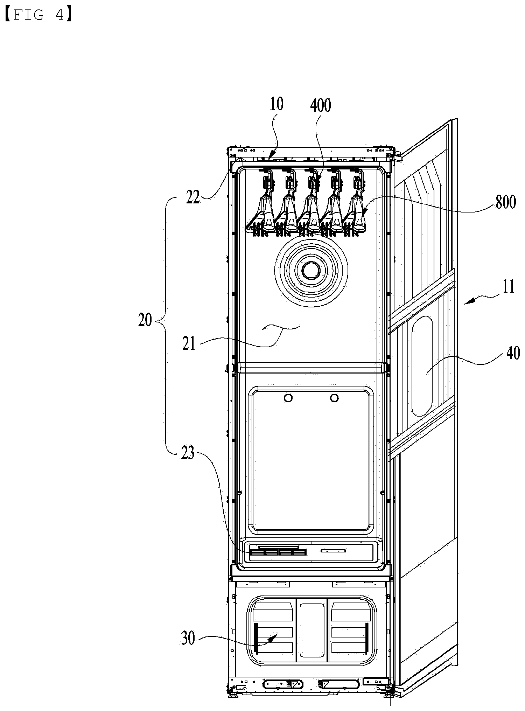

FIG. 4 is a view illustrating one example of a laundry treatment apparatus 1 according to the present disclosure.

Referring to FIG. 4, the laundry treatment apparatus according to the present disclosure may include a cabinet 10 having an opening at a front side thereof, a door 11 rotatably provided in the cabinet 10 to open and close the opening, an inner case 200 provided in the cabinet to provide an accommodation space in which clothes (L) are accommodated, a machine room 300 communicating with the inner case 200 so as to generate at least one of air (hot air) and moisture supplied to an accommodation space 21, and a holder 400 provided in at least one of the inner case 20 and the door 11 such that the clothes (L) are disposed in or fixed to the accommodation space.

The cabinet 10 may be provided to form exterior appearance of the laundry treatment apparatus, and the height of the cabinet 10 may be greater than the width of the cabinet 10. Accordingly, clothes (L) having a long length such as pants (trousers) can be disposed in the accommodation space 21 without being folded, so that it is possible to prevent wrinkles from occurring in the clothes (L).

The cabinet 10 may be formed of a metal material, and may also be formed of a resin material such as reinforced plastic in so far as the strength of the cabinet 10 can be maintained.

The inner case 20 may be provided inside the cabinet 10, and may be formed in a case shape forming the accommodation space 21.

The inner case 20 may be made of a material capable of maintaining strength without being deformed or undergoing chemical reaction with foreign substances discharged from the clothes (L) or by hot air or moisture supplied from the supply unit. For example, the inner case 20 may be made of styrene resin series such as ABS, ASA, or the like.

The inner case 20 may be provided to communicate with the machine room 30 to receive heated air or moisture from the machine room 30 or to discharge air to the machine room 30. To this end, the inner case 20 may include a plurality of through-holes 23 communicating with the machine room 30.

The machine room 30 may be provided to be separated from the accommodation space 21 within the cabinet 10.

The machine room 30 may be disposed below the accommodation space 21 to supply hot air or steam having a relatively lower density than air to the accommodation space 21.

In other words, the air supplied to the accommodation space 21 by the machine room 30 may be heated air (hot air), and the moisture supplied to the accommodation space 21 by the supply unit may be steam. As a result, as long as the machine room 30 is disposed below the accommodation space 21, hot air or steam can be evenly supplied into the accommodation space 21 without a separate blower.

Although not shown in the drawings, the machine room 30 may include a circulation duct communicating with the inner case 20, and a heat pump disposed inside the circulation duct to dehumidify and heat the air circulating in the circulation duct. The heat pump may include an evaporator and a condenser disposed on the basis of the direction of air circulation, a compressor for compressing the refrigerant discharged from the evaporator and supplying the compressed refrigerant to the condenser, and an expansion valve for expanding and cooling the refrigerant passing through the condenser.

In addition, the circulation duct may communicate with a steam supply unit for supplying steam. The steam supply unit may include a water container mounted in the machine room 30, a conversion unit for converting water stored in the water container into steam by heating or vibrating the water, and a steam pipe through which steam having passed through the conversion unit can communicate with a circulation duct.

Since the above-described configuration may be the same as that of the conventional laundry treatment apparatus of FIG. 1 or 2, a detailed description thereof will herein be omitted for brevity.

Meanwhile, the door 11 may be rotatably coupled to the cabinet 10 to open and close the opening. The door 11 may be provided to shield not only the inner case 20 but also the front of the machine room 30.

Accordingly, not only can hot air or moisture supplied to the accommodation space 21 be prevented from leaking outside, but also heat generated in the machine room 30 can be prevented from being transferred to the outside.

Since the door 11 is provided to open and close the front of the inner case 20, the inner surfaces of the inner case 20 and the inner surface of the door 11 may form the outer surface of the accommodation space 21.

The holder 400 may be provided to hold the clothes L in the accommodation space 21 inside the inner case 20.

The holder 400 may be provided at a top surface 22 of the inner case 20 or may be provided at an upper part of the side surface of the inner case 20 such that the clothes (L) can be held in the accommodation space 21 in a fully unfolded state in which the clothes (L) are unfolded.

Thus, the holder 400 may allow the clothes (L) to be held in the accommodation space 21 in an unfolded state due to gravity, so that the clothes (L) can be evenly exposed to air or moisture supplied from the machine room 30.

The laundry treatment apparatus according to the present disclosure may further include a pressing unit 40 coupled to the door 11 to press the clothes.

The pressing unit 40 may press the clothes (L) in a state in which the clothes (L) are hung on the door 11. Accordingly, the pressing unit 40 can remove wrinkles from the clothes (L) or can form desired creases in the clothes (L).

Meanwhile, the laundry treatment apparatus according to the present disclosure may further include a drive unit 200 provided to shake the holder 400.

The fact that the drive unit 200 shakes the holder 400 means that the drive unit 200 vibrates or reciprocates the holder 400 or performs pendulum motion of the holder 400. When the holder 400 is shaken, the clothes hanger mounted on the holder 400 is also shaken to move clothes hung thereon.

When the holder 400 is shaken, the clothes hanger 800 mounted on the holder 400 is also shaken, so that clothes hung on the clothes hanger 800 can be dusted off.

Moreover, when the clothes hanger 800 is shaken due to shaking of the holder 400, the clothes move inside the inner case 20 to be more evenly exposed to hot air or steam supplied into the accommodation space 21, thereby maximizing refresh performance of the clothes.

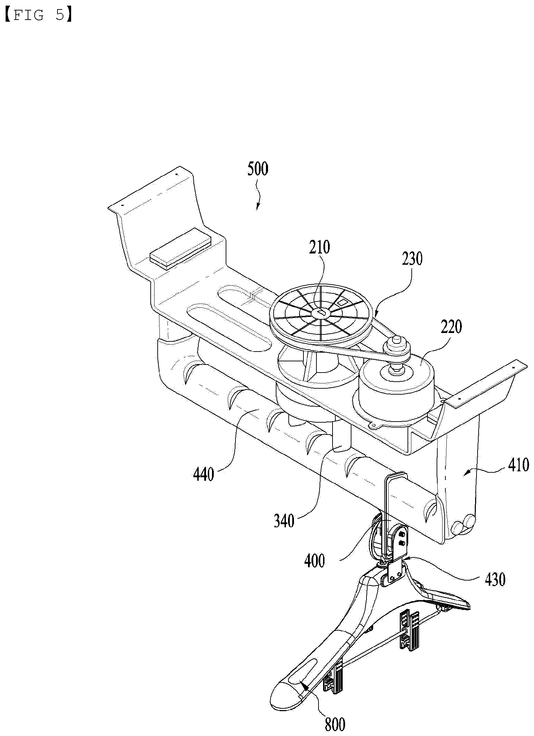

FIG. 5 is a view illustrating an example of the drive unit of the laundry treatment apparatus according to an embodiment of the present disclosure.

Referring to FIG. 5, the laundry treatment apparatus according to the present disclosure may include a support 500 that can be fixed to the cabinet 10, and a drive unit 200 seated in the support 500.

The drive unit 200 may include a motor 220 seated in the support 500, a rotary shaft 210 rotated by the motor 220, and a rotary coupling unit 230 for transferring rotational force of the motor 220 to the rotary shaft 210.

A lower portion of the drive unit 200 may include a power transmission unit 300 that converts rotational motion of the rotary shaft 210 into rectilinear reciprocating motion. The power transmission unit 300 may include a cam member configured to rotate by coupling to the rotary shaft 210, and a rectilinear conversion unit 340 configured to rectilinearly move by the cam member.

A lower end of the support 500 may include a holder 400 for holding the clothes hanger 800 thereon.

The holder 400 may include a holding plate 410 coupled to lower portions of both sides of the support 500 so that a lower end thereof can be shaken horizontally, and a support bar 440 supported by the holding plate 410.

The support bar 440 may be coupled to the rectilinear conversion unit 340 and can be shaken from side to side by the rectilinear conversion unit 340.

The clothes hanger 800 is mounted on the support bar 440, so that the clothes hanger 800 can vibrate and shake from side to side by the support bar 440.

In this case, the holder 400 may further include a hanger support 430 for supporting a main body of the clothes hanger 800.

The hanger support 430 may be designed to be in surface contact with one or both surfaces of the clothes hanger 800, and may be coupled to the holding plate 410 or disposed below the holding plate 410.

The hanger support 430 may be provided to move integrally with the holding plate 410 or the support bar 440. Accordingly, the hanger support 430 may induce the clothes hanger 800 to move integrally with the holder 400 while supporting at least one surface of the clothes hanger 800.

By the hanger support 430, the clothes hanger 800 may move together with the holder 400 while overcoming inertial force thereof.

The amplitude or direction of the clothes hanger 800 shaking in the holder 400 may be limited to a certain level or less by the hanger support 430. For example, the relative motion of the clothes hanger 800 in the holder 400 may be restricted by the hanger support 430.

Accordingly, even if the plurality of clothes hangers 800 is provided in the laundry treatment apparatus, the clothes hangers 800 can be prevented from colliding with each other by simultaneously moving with the same amplitude in the same direction.

In addition, since the plurality of clothes hangers 800 is guided to move integrally by the hanger support 430, collision of clothes mounted on the clothes hangers 800 can be prevented.

In addition, each of the clothes hangers 800 is restricted from arbitrarily moving in the holder 400 by the hanger support 430, so that stability of the drive unit 200 and the holder 400 can be guaranteed.

Although the hanger support 430 is shown as supporting only one side of the clothes hanger 800 in the drawings, the scope or spirit of the present disclosure is not limited thereto, and the hanger support 430 may also be provided to support both sides of the clothes hanger 800 as needed.

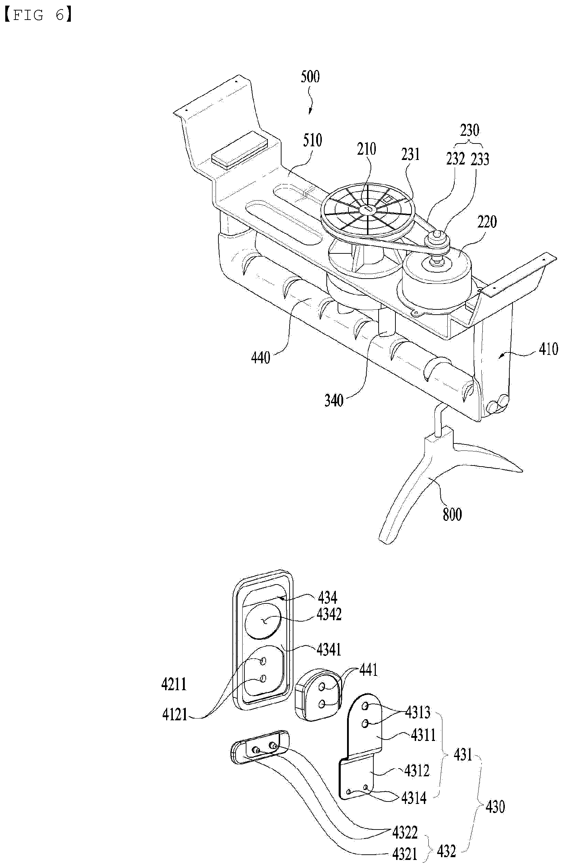

FIG. 6 is a view illustrating one example of the hanger support 430.

Referring to FIG. 6, the holder 400 may include a fixing plate 414 coupled to the support bar 440 to support a load of the clothes hanger 800.

The fixing plate 414 may include a fixing body 4141 that is fixed to the support bar 440 and moves integrally with the support bar 440, and a fixing groove 4142 formed in the support bar 440 by penetrating the fixing body 4141. The fixing groove 4142 may be formed in a shape corresponding to a cross section of the support bar 440, and may be coupled to the support bar 440 in various ways such as interference fit.

The fixing body 4141 may be coupled with a hanger unit 420 on which a hook of each clothes hanger 800 is mounted, and may also be coupled to an intermediary body 441 to which a fastening member capable of coupling the hanger unit 420 to the fixing body 4141 is fixed.

The hanger support 430 may be coupled to the fixing body 4141 such that the hanger support 430 can support at least one surface of the clothes hanger 800 while being in surface contact with the at least one surface of the clothes hanger 800.

The hanger support 430 may include a support body extending farther downward than the hanger unit 420 by connecting to the holding plate, and a support member 432 coupled to one surface of the clothes hanger 800 by connecting to the support body 431.

The support body 431 may be formed of a metal plate to ensure bonding strength and durability with the holding plate 410, and may prevent deformation thereof even when a load is transmitted to the clothes hanger 800.

The support member 432 may be provided as an elastic member and may be in surface contact with one surface of the clothes hanger 800. The support member 432 may increase contact force with the clothes hanger 800, and may prevent damage to the clothes hanger 800 by generating a force capable of moving the clothes hanger 800.

The support body 431 may be formed to extend such that an upper end thereof is coupled to the fixing plate 414 and the other end thereof is arranged to face one surface of the main body of the clothes hanger 800.

The support body 431 may include a first body 4311 coupled to one surface of the fixing plate 414, and a second body 4312 extending downward from the hanger unit 420 in the first body 4311.

The second body 4312 may be bent toward the hanger unit 420 or the clothes hanger 800 such that the second body 4312 can be disposed closer to the clothes hanger 800.

The first body 4311 may include a body hole 4313 through which the fastening member 450 can pass. The body hole 4313 may be implemented as a plurality of body holes as necessary.

The support member 432 may be coupled to the support body 431 to support one surface of the clothes hanger. The support member 432 may be provided as an elastic member, and may be coupled to and fixed to the second body 4312.

The support member 432 may be larger in width than the second body 4312, so that one surface of the clothes hanger 800 can be supported more reliably.

The second body 4312 may be provided with a second body hole 4314 coupled to the support member 432.

The support member 432 may include a protrusion 4322 inserted into and coupled to the second body hole 4314.

Of course, the support member 432 may be coupled to the second body 4312 by a separate fastening member.

The support member 432 may be provided with a recessed surface that is recessed according to the shape of the second body 4312 so as to be fixed in close contact with the second body 4312. The protrusion 4322 may protrude from the recessed surface.

The first body 4311 may be disposed at a height where the first body 4311 partially overlaps the hanger unit 420. Therefore, the first body can be coupled to the holding plate 410 together with the hanger unit 420 through one fastening member.

Accordingly, the clothes hanger 800 may be supported by the hanger support 430 so that the clothes hanger 800 can move integrally with the holder 400.

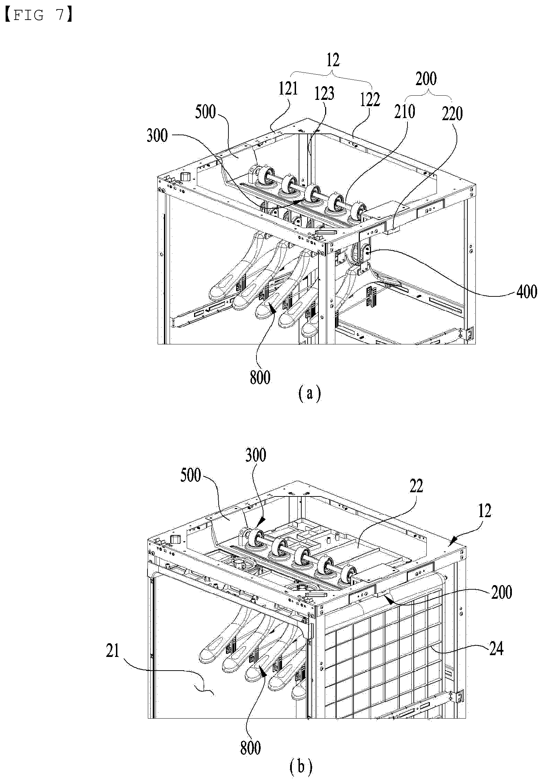

FIG. 7 is a view illustrating another example of the drive unit of the laundry treatment apparatus.

Referring to FIG. 7(a), the cabinet 10 may further include a frame 12 supporting a panel disposed outside the cabinet 10.

The frame 12 may be provided in a rectangular parallelepiped shape, such that the exterior of the frame 12 is coupled to a panel of the cabinet 10 and the interior of the frame 12 is configured to support the inner case 20.

Specifically, the frame 12 may include a first frame 121 disposed in forward and backward directions, a second frame 122 coupled to the first frame 12 and disposed in the width direction, and a third frame 123 coupled to the first frame 121 or the second frame 122 and disposed in the height direction.

The first frame 121, the second frame 122, and the third frame 123 may be combined to form a body frame formed in a rectangular parallelepiped shape.

The drive unit 200 may include a motor 220 coupled to and supported by the frame 12 to generate power, and a rotary shaft 210 provided to rotate by the motor 220.

The rotary shaft 210 may be disposed in the width direction of the inner case 20, and the motor 220 may be fixed to the frame 12 to generate power for rotating the rotary shaft 210.

Meanwhile, the rotary shaft 210 may be provided to transmit power for shaking the holder 400 while being rotated by the motor 220.

In addition, the holder 400 may be provided as a plurality of holders 400, the holders 400 may be spaced apart from each other by a predetermined distance in the longitudinal direction of the rotary shaft 210.

The rotary shaft 210 may be provided to shake all of the holders 400 while supporting all of the holders 400.

Accordingly, the laundry treatment apparatus 1 according to the present disclosure can be designed such that the plurality of holders 400 can be simultaneously shaken by one motor 220 and one rotary shaft 210.

Meanwhile, since the rotary shaft 210 is configured to rotate and is disposed in the height direction inside the inner case 20, the rotary shaft 210 may have difficulty in directly supporting the holder 400 on which clothes or the hangers are hung.

Accordingly, the laundry treatment apparatus 1 according to the present disclosure may further include a power transmission unit 300 capable of shaking the holder 400 by transmitting power of the rotary shaft 210 to the holder 400.

The power transmission unit 300 may be coupled to the rotary shaft 210 to support the holder 400. The power transmission unit 300 may connect the rotary shaft 210 to the holder 400, and at the same time may transmit the load of the holder 400 to at least a portion of the rotary shaft 210 so that rotational power of the rotary shaft 210 can be transmitted to the holder 400.

Meanwhile, since the holder 400 is supported by the rotary shaft 210 through the power transmission unit 300, the rotary shaft 210 need not be directly exposed to the inside of the inner case 20.

In other words, the rotary shaft 210 may be disposed outside the inner case 20, and the power transmission unit 300 coupled to the rotary 210 may be coupled to the holder 400 by extending into the inner case 20.

In addition, the inner case 20 may be disposed outside the inner case, and the holder 400 may be formed to extend by penetrating the inner case 20 so that the holder 400 can be coupled to the power transmission unit 300 coupled to the rotary shaft 210.

In any case, since the rotary shaft 210 is disposed outside the inner case 20, the rotary shaft 210 can be blocked from being exposed to the inside of the inner case 20.

In addition, the motor 220 and the rotary shaft 210 may also be directly supported by the inner case 20.

However, since the inner case 20 is formed of resin, rigidity or durability of the inner case 20 may be insufficient to directly support the motor 220 and the rotary shaft 210.

Therefore, the laundry treatment apparatus according to the present disclosure may further include a support 500 for supporting the motor 220 and the rotary shaft 210.

For example, the support 500 may be provided to support the drive unit 200 while being coupled to and supported by the frame 12. In addition, the support 500 may be seated in and supported by the first frame 121 and the second frame 122, and the motor 220 may be fixed and supported by connecting to the support 500.

The support 500 may be formed of a metal material that is supported by the case 10 or the inner case 20 and supports the rotary shaft 210 or the motor 220.

When at least a portion of the support 500 is disposed at a lower position than the rotary shaft 210, the rotary shaft 210 can be prevented from being exposed outside or can be minimally exposed outside.

At least a portion of the support 500 may be provided to shield the rotary shaft 210.

As a result, the laundry treatment apparatus 1 according to the present disclosure can provide power needed to shake the holder 400, and the rotary shaft 210 disposed in the width direction of the inner case can be prevented from being exposed outside or can be exposed outside in a restricted manner.

Thus, the rotary shaft 210 and the motor 220 can be prevented from being exposed to the inside of the inner case 20.

As a result, the rotary shaft 210 and the motor 220 may be blocked from being exposed to hot air or steam supplied to the inside of the inner case 20, so that corrosion, short-circuiting, etc. of the rotary shaft 210 and the motor 220 can be prevented, and heating of the rotary shaft 210 and the motor 220 can also be prevented.

Therefore, the laundry treatment apparatus can prevent the rotary shaft 210 from being bent downward or damaged by the user.

Moreover, the laundry treatment apparatus can minimize contact between the user's body and the rotary shaft 210 or can prevent the user's body from coming into contact with the rotary shaft 210, thereby preventing occurrence of accidents in which the user's body is injured by the rotary shaft 210 or burned by the overheated rotary shaft 210.

In addition, since the drive unit 200 and the power transmission unit 300 are disposed outside the inner case 20, the laundry treatment apparatus can completely block the clothes or the hanger from contacting the power transmission unit 300 or the inner case 20.

Meanwhile, at least a portion of the power transmission unit 300 may be disposed above the support 500. As a result, a portion of the power transmission unit 300 may also be supported by the support 500.

Therefore, the load to be applied to the rotary shaft 210 may be dispersed through the support 500, and thus bending of the rotary shaft 210 can be prevented. Therefore, continuous rotation of the rotary shaft 210 can be ensured so that performance of the laundry treatment apparatus shaking the clothes can be maintained.

Referring to FIG. 7(b), the rotary shaft 210 and the motor 220 may be disposed outside the inner case 20. The rotary shaft 210 and the motor 220 may be supported by the support 500 and disposed above the top surface 22 of the inner case 20.

Thus, exposing the rotary shaft 210 or the motor 220 to hot air or steam supplied to the accommodation space 21 of the inner case 20 can be minimized or prevented.

Therefore, short circuit of the motor 220 can be prevented, and corrosion or heating of the rotary shaft 210 or the motor 210 can also be prevented.

The drive unit 200 is disposed outside the inner case 20, and the holder 400 is exposed inside the inner case 20 so that the clothes hanger 800 can be mounted thereon. Therefore, the inner case 20 may further include a through-hole 23 through which the holder 400 or the power transmission unit 300 can pass.

At least a portion of the through-hole 23 may be shielded by the support 500, or may be shielded by a sealing unit 700 to be described later.

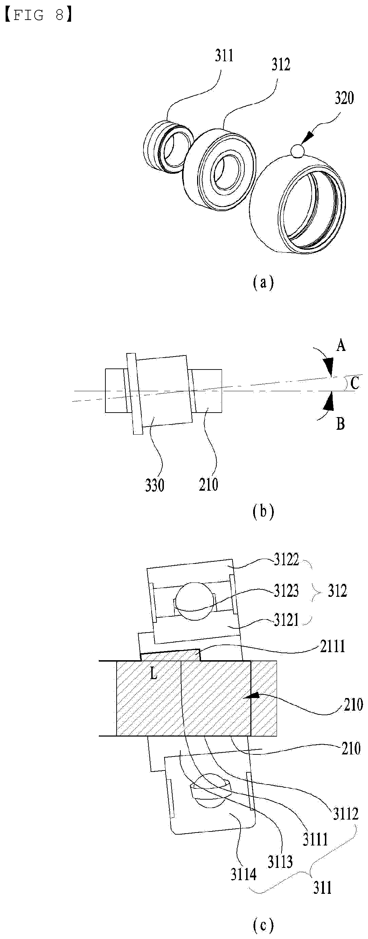

FIG. 8 is a view illustrating an example of the power transmission unit of the laundry treatment apparatus according to the present disclosure.

Referring to FIG. 8, the power transmission unit 300 may support the holder 400, and may allow the holder 400 to wobble (or sway), thereby moving the holder 400 back and forth (i.e., pendulum motion).

The pendulum motion may refer to a motion in which the direction of motion does not change rapidly or even when the direction of motion is changed, the direction of motion changes gradually unlike rectilinear reciprocating motion.

The power transmission unit 300 is coupled to the rotary shaft 210 in a manner that the rotary shaft 210 can penetrate the power transmission unit 300. When the rotary shaft 210 rotates, the shaft is changeable by power of the rotary shaft 210 so that the holder 400 can move back and forth (i.e., pendulum motion).

Referring to FIG. 8(a), the power transmission unit 300 may include a bearing unit 310 and a rigid body unit 320. In more detail, an inner circumferential surface of the bearing unit 310 is coupled to the rotary shaft 210 so that the bearing unit 310 can rotate, and an outer circumferential surface of the bearing unit 310 can rotate independently of the inner circumferential surface of the bearing unit 310. The rigid body unit 320 is coupled to the bearing unit 310 such that the axial direction of the rigid body unit 320 can be changed together with the bearing unit 310.

Thus, the bearing unit 310 may rotate together with the rotary shaft 210, but the rigid body unit 320 may be prevented from rotating together with the rotary shaft 210. Accordingly, the holder 400 may be extended or coupled to the rigid body unit 320 such that the holder 400 does not rotate with the rotary shaft 210 and can allow the clothes hanger 800 to be held in the inner case 20.

The rigid body unit 320 may also be provided in the form of a cam or the like such that the position of the rigid body unit 320 is variable when the rotary shaft 210 rotates.

However, when the rotary shaft 210 rotates, the rigid body unit 320 may move in a so-called wobble plate motion in which only the axial direction of the rotary shaft 210 is changed.

Accordingly, the holder 400 coupled to the rigid body unit 320 moves in a manner that the axial direction thereof is changed so that the holder 400 can also move in a wobble plate motion or in a pendulum motion.

To this end, the rigid body unit 320 may be disposed inclined with respect to the rotary shaft 210, the bearing unit 310 may be provided obliquely, and the rigid body unit 320 may accommodate the bearing unit 310 and may move integrally with the bearing unit 310.

The bearing unit 310 may be provided such that an inner ring is coupled in parallel to the rotary shaft 210 and an outer ring is disposed inclined with respect to the rotary shaft 210, such that the central axis corresponding to the outer ring is subjected to precession based on the rotary shaft 210.

However, in order to apply the existing bearing structure to the laundry treatment apparatus, the bearing unit 310 according to the present disclosure may include an inclined pipe 311 and a rotary bearing 312. The inclined pipe 311 may be coupled to the rotary shaft 210 to rotate together with the rotary shaft 210 such that the outer circumferential surface of the inclined pipe 311 is disposed inclined with respect to the rotary shaft 210. The rotary bearing 312 may be coupled to accommodate the inclined pipe 311.

The rotary bearing 312 may be provided as a general bearing in which inner and outer rings may be arranged parallel to each other, and the rigid body unit 320 may be coupled to and fixed to the outer ring of the rotary bearing 312. Therefore, it is not necessary to separately manufacture the structure of the rotary bearing 312, resulting in reduction in production costs.

The rigid body unit 320 may be provided in a cylindrical or ring shape with a much larger diameter than a height.

As the diameter of the rigid body unit 320 increases, the movement of the holder 400 coupled to the rigid body unit 320 increases, so that the shaking effect (also called the dusting effect) of the clothes may increase.

The rigid body unit 320 may be provided with a much larger diameter than a thickness of the axial direction.

Referring to FIG. 8(b), the inclined pipe 311 may be coupled to the rotary shaft 210 while accommodating the rotary shaft 210.

If the axial direction or the extension direction of the rotary shaft 210 is defined as a reference-axis direction (B), and if the axial direction of the outer circumferential surface of the inclined pipe 311 is defined as an inclined-axis direction (A), the inclined-axis direction (A) may be coupled to the reference-axis direction (B) in a manner that the inclined-axis direction (A) is disposed to have an inclined angle (C) with respect to the reference-axis direction (B).

Therefore, whereas the inclined pipe 311 is coupled to the rotary shaft 210 and rotates integrally with the rotary shaft 210, the inclined pipe 311 rotates with the rotary shaft 210 while being twisted by the inclination angle (C) with respect to the rotary shaft 210, so that the inclined pipe 311 may be subjected to precession with respect to the rotary shaft 210.

Referring to FIG. 8(c), the inner circumferential surface 3112 of the inclined pipe 311 may be arranged parallel to the rotary shaft 210, and the outer circumferential surface 3113 of the inclined pipe 311 may be inclined by an inclination angle (C) with respect to either the inner circumferential surface of the inclined pipe 311 or the rotary shaft 210.

However, the outer circumferential surface 3113 of the inclined pipe 311 may be formed in a cylindrical shape.

As a result, when the rotary bearing 312 is obliquely coupled to the outer circumferential surface 3113 of the inclined pipe 311, the rotary bearing 312 is subjected to precession by the inclined angle (C) with respect to the rotary shaft 210. In addition, when the rigid body unit 320 is coupled to the rotary bearing 312, the axial direction of the rigid body unit 320 can be changed together with the axial direction of the inclined pipe 311 and the axial direction of the rotary bearing 312.

The rotary bearing 312 may include an inner ring 3121 configured to accommodate the outer circumferential surface of the inclined pipe 311, an outer ring 3122 accommodated in the rigid body unit 320, and a rotation member 3123 provided to rotate the inner ring 3121 and the outer ring 3122 independently of each other.

The rotation member 3123 may include a plurality of balls that rotates while simultaneously contacting the inner ring 3121 and the outer ring 3122, without being limited thereto. As long as the rotation member 3123 can rotate the inner ring 3121 and the outer ring 3122 independently of each other, the rotation member 3123 may also be provided in any structure such as fluid, a magnet, and the like.

The inner ring 3121 may be coupled to the outer circumferential surface 3113 of the inclined pipe 311 and rotated together with the outer circumferential surface 3113, without being limited thereto. If necessary, the outer ring 3122 may not rotate together with the inner ring 3121 due to movement of the rotation member 3123.

Accordingly, since the outer ring 3122 is coupled to the rigid body unit 320 and the rigid body unit 320 supports the load of the holder 400, the outer ring 3122 may not rotate even when the rotary shaft 210 and the inner ring 3121 of the rotary bearing 3121 rotate.

As a result, the outer ring 3122 may be provided in a manner that only the axial direction thereof is changed when the inner ring 3121 precesses together with the inclined pipe 311, and the rigid body unit 320 may also wobble, changing the direction of the central axis thereof.

On the other hand, in order to strengthen the coupling force with the rotary shaft 210, the inclined pipe 311 may be provided to accommodate at least a portion of the outer circumferential surface of the rotary shaft 210 or may be provided to be inserted into the outer circumferential surface of the rotary shaft 210.

For example, the rotary shaft 210 may include a protrusion 2111 protruding from the outer circumferential surface to be inserted into the inclined pipe 311. The inclined pipe 311 may include an inclined groove 3111 that can be formed to include the protrusion 2111 on the inner circumferential surface.

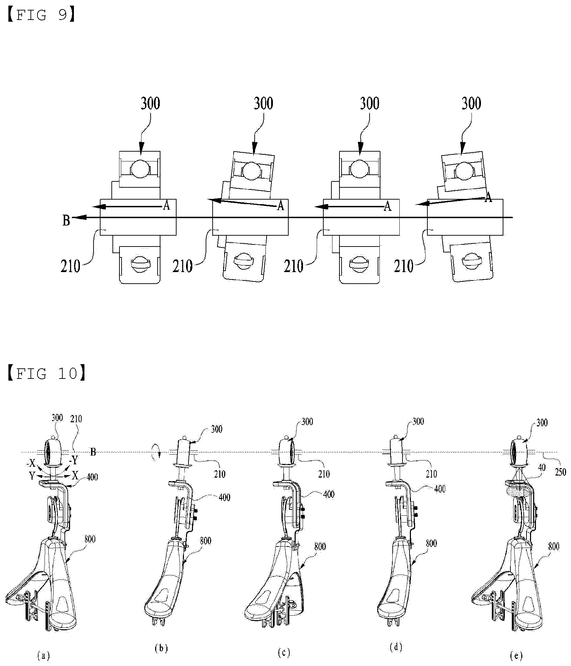

FIG. 9 is a view illustrating a process for driving the power transmission unit of the laundry treatment apparatus according to the present disclosure.

Referring to FIG. 9(a), the inclined pipe 311 may be disposed inclined by an inclination angle (C) with respect to the rotary shaft 210 when viewed upward from the ground. As a result, when viewed from a cross-section of the inclined pipe 311, the reference-axis direction (A) and the inclined-axis direction (B) may be arranged as if they were parallel to each other.

However, since the axial direction of the bearing unit 310 is twisted, the left surface of the bearing unit 310 may be exposed and the right surface of the bearing unit 310 may be invisible.

Referring to FIG. 9(b), when the inclined pipe 311 is rotated 90 degrees (90°) along with the rotary shaft 210, the inclined-axis direction (A) of the inclined pipe 311 may be disposed to face upward by the inclination angle (C) of the inclined pipe 311.

Referring to FIG. 9(c), when the inclined pipe 311 is additionally rotated 90 degrees (90°) along with the rotary shaft 210, the inclined pipe 311 is disposed to be inclined by the inclination angle (C) with respect to the rotary shaft 210 in a direction in which the inclined pipe 311 moves downward from the ground. As a result, when viewed from a cross-section of the inclined pipe 311, the reference-axis direction (A) and the inclined-axis direction (B) may be arranged as if they were parallel to each other.

However, since the axial direction of the bearing unit 310 is twisted, the right surface of the bearing unit 310 may be exposed and the left surface of the bearing unit 310 may be invisible.

Referring to FIG. 9(d), when the inclined pipe 311 rotates additionally by 90 degrees (90°) along with the rotary shaft 210, the inclined-axis direction (A) of the inclined pipe 311 may be disposed such that the inclined-axis direction (A) of the inclined pipe 311 faces downward by the inclination angle (C) of the inclined pipe 311.

When the inclined pipe 311 rotates together with the rotary shaft 210, the inclined pipe 311 can process by the inclination angle (C) of the inclined pipe 311, and the bearing unit 310 may rotate in a direction corresponding to rotation of the inclined pipe 311.

Although the bearing unit 310 serving as one configuration is structured in a manner that the outer ring is disposed inclined with respect to the inner ring, the bearing unit 310 can move while the axial direction thereof is changed as shown in the drawings.

As a result, the inclined pipe 311 may rotate in an axial direction while tracing a conical path with respect to the rotary shaft 210.

Accordingly, the rigid body unit 320 coupled to the inclined pipe 311 can also receive the axially rotating force while tracing a conical route in the axial direction, and the holder 400 coupled to the rigid body unit 320 can also perform rotational motion or pendulum motion while tracing the conical route.

In other words, the bearing unit 310 may process with respect to the rotary shaft 210, and the outer ring of the bearing unit 310 may be changed as if the axial direction of the bearing unit 310 has rotated while tracing a conical route based on the rotary shaft 210.

Since the rigid body unit 320 is fixed to the outer ring of the bearing unit 310 (e.g., the outer ring of the rotary bearing 312), the axial direction of the rigid body unit 320 is changed in the same manner as in the axial direction of the bearing unit 310.

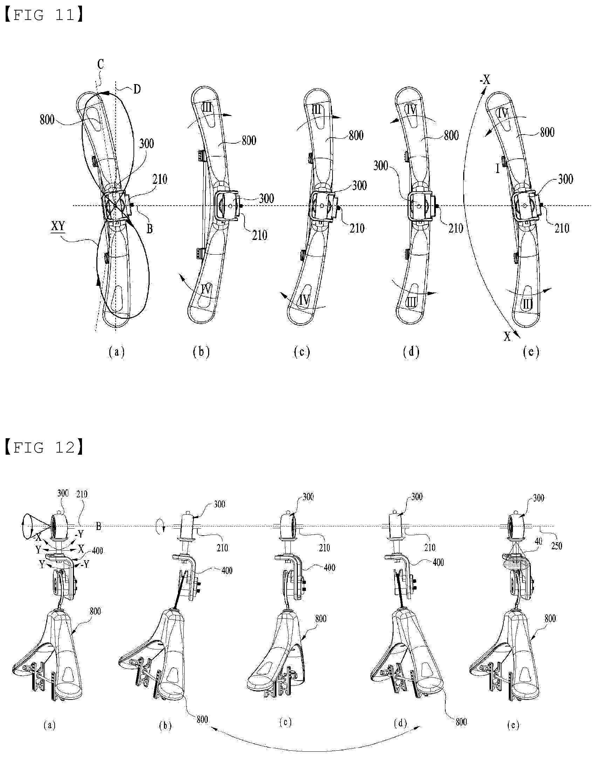

FIGS. 10 and 11 are views illustrating states of the clothes hangers moving by the power transmission unit of the laundry treatment apparatus according to the present disclosure.

Referring to FIGS. 10(a) and 11(a), the power transmission unit 300 may be obliquely coupled to the rotary shaft 210 such that the power transmission unit 300 can move in a manner that the axial direction thereof is twisted.

The holder 400 may be disposed at a lower position than the rotary shaft 210, and may extend toward the bottom surface of the inner case 20.

The holder 400 is coupled to the power transmission unit 300 and can move integrally with the power transmission unit 300. Since the clothes hanger 800 is seated on the holder 400, the clothes hanger 800 can also move integrally with the power transmission unit 300.

As a result, when the axial direction of the power transmission unit 300 is moved to be changed, the holder 400 and the clothes hanger 800 can also move integrally in correspondence with such axial movement of the power transmission unit 300.

Assuming that the direction in which the rotary shaft is arranged is referred to as a Y-axis direction and the forward and backward direction is referred to as an X-axis direction, the state of FIG. 10(a) may be considered to be a situation in which the left surface of the power transmission unit 300 is disposed inclined toward the (+X+Y) directions. Therefore, the holder 400 and the clothes hanger 800 may also be disposed inclined toward the (+X+Y) directions.

Referring to FIGS. 10(b) and 11(b), the rotary shaft 210 rotates such that the axial direction of the power transmission unit 300 may be twisted upward on the Y-axis.

As a result, the holder 400 and the clothes hanger 800 may also move to be inclined toward the upper left side. That is, the holder 400 and the clothes hanger 800 may be lifted upward by an inclination angle (C) while facing the XY direction and then turning to face the Y-axis direction.

Referring to FIGS. 10(c) and 11(c), the rotary shaft 210 may be further rotated so that the axial direction of the power transmission unit 800 is inclined toward the (-X+Y) direction.

As a result, the holder 400 and the clothes hanger 800 may be disposed inclined so that their right sides face forward. That is, the holder 400 and the clothes hanger 800 may be arranged in the Y-axis direction and then twisted in the (-X+Y) direction, so that the holder 400 and the clothes hanger 800 can move downward as much as the inclination angle.

Referring to FIGS. 10(d) and 11(d), the rotary shaft 210 may be further rotated so that the axial direction of the power transmission unit may be inclined downward on the Y-axis.

As a result, the holder 400 and the clothes hanger 800 may also be disposed inclined downward on the Y-axis. That is, the holder 400 and the clothes hanger 800 may be twisted while facing the (-X+Y) direction and then moving downward on the Y-axis, so that the holder 400 and the clothes hanger 800 can further move downward by the inclination angle.

Referring to FIGS. 10(e) and 11(c), when the rotary shaft 210 further rotates a total of 360 degrees (360°), the resultant state of the rotary shaft 210 may return to the state of FIG. 10(a). That is, the left side of the power transmission unit 300 may be disposed inclined toward the (+X+Y) direction, and the holder 400 and the clothes hanger 800 may also be disposed inclined toward the (+X+Y) direction, so that the holder 400 and the clothes hanger 800 can move upward by the inclination angle.

To sum up, the axial direction of the power transmission unit 300 may rotate while tracing a conical route with respect to the rotary shaft 210. As a result, the power transmission unit may reciprocate by the inclination angle (C) in the forward-and-backward direction and in the up-and-down direction, respectively.

Specifically, the holder 400 may be coupled to the rigid body unit 320 so as to move integrally with the rigid body unit 320. In addition, the clothes hanger 800 is also coupled to the holder 400 so that the clothes hanger can move integrally with the rigid body unit 320.

As a result, each of the holder 400 and the clothes hanger 800 may also reciprocate by the inclination angle (C) in the vertical direction, and may also reciprocate by the inclination angle (C) in the forward and backward directions.

As a result, when the axial direction of the rigid body 310 is changed as if the rigid body 310 were axially precessing with respect to the rotary shaft, the left or right side of the holder 400 and the clothes hanger 800 may repeatedly move upward or downward, such that one arm or the other arm of the clothes hanger 800 can move to repeatedly face left and right.

As a result, the clothes hung on the clothes hanger 800 may reciprocate to repeatedly face upward and downward with respect to an arbitrary surface, and may repeatedly reciprocate to be twisted in forward and backward directions with respect to the arbitrary surface.

Accordingly, since all of the clothing is twisted in various directions of the inner case 20, all of the clothing can be evenly exposed to hot air or steam supplied to the accommodation space 21.

Since the holder 400 extends from the rigid body 310 toward the bottom surface of the inner case 20, the holder 400 can serve as a rotation axis of a pendulum motion. Thus, the holder 400 and the clothes hanger 800 can perform the pendulum motion.

Specifically, as the axial direction of the power transmission unit 300 rotates in a conical shape based on the Y-axis, the holder 400 and the clothes hanger 800 can receive the rotational force generated while moving in a conical shape with respect to the height direction.

In addition, the holder 400 and the clothes hanger 800 may be rotated at any time in the XY-axis direction, and may be pendulum-moved in the (+Y)-axis and (-Y)-axis directions whenever the rotary shaft 210 rotates.

Furthermore, the holder 400 and the clothes hanger 800 may also be provided to pendulum-move in the (+X)-axis and (-X)-axis directions whenever receiving the conically rotating force.

For this reason, the holder 400 and the clothes hanger 800 may perform a pendulum motion based on the X-axis direction while being twisted, and may also perform a pendulum motion based on the Y-axis direction. Therefore, the holder 400 and the clothes hanger 800 may perform a pendulum motion or a rotational motion in a three-dimensional (3D) manner, so that the effect of dusting off the clothes in the vertical direction can be obtained.

In this pendulum motion, the direction of motion does not change rapidly at a specific time point such as forward and backward reciprocating movements. That is, such a pendulum motion may have a constant change in momentum at all the time points, or may have a very small change in momentum at all the time points. Therefore, since the change in momentum of the holder 400 and the clothes hanger 800 coupled to the rigid body 310 is constant at all the time points, strong inertial force may not occur at a specific time point. Therefore, since sudden inertial force does not occur in the entirety of the power transmission unit 300, the holder 400, and the clothes hanger 800, noise or vibration generated in the laundry treatment apparatus 1 can be greatly reduced, and the durability of the drive unit 200, the power transmission unit 300, the holder 400, and the clothes hanger 800 can be guaranteed.

In addition, since the clothes hung on the clothes hanger 800 have a constant change in momentum or perform a gentle pendulum motion, the laundry treatment apparatus can prevent the clothes from suddenly colliding with the inner case 20 or the like or can prevent the clothes from colliding with each other, thereby preventing damage to the clothes.

On the other hand, referring to FIG. 11(a), the rigid body unit 320 may not strictly move integrally with the bearing unit 310. In other words, even if the bearing unit 310 rotates in the axial direction based on the rotary shaft 210, since the rigid body unit 320 rotatably accommodates the bearing 310, the rigid body unit 320 can move independently of the bearing unit 310.

That is, the rigid body unit 320 does not move exactly integrally with the bearing unit 310 due to loads of the holder 400, the clothes hanger 800, and the clothes, and may receive inertial force to stay in place.

Therefore, even if the bearing unit 310 is axially rotated in a conical shape, the rigid body unit 320 can rotate along the outer circumferential surface of the bearing unit 310, such that the lower portion of the rigid body unit 320 and the holder 400 may rotate while tracing a figure-of-8 route.

That is, whenever the rotary shaft 210 rotates once, the bearing unit 310 also rotates once in a circle in the axial direction based on the rotary shaft. However, the movement of the rigid body unit 320 is restricted due to the load of the holder 400 and the clothes hanger 800, such that the rigid body unit 320 can perform the pendulum motion while tracing the figure-8 route.

In other words, the rigid body unit 320 can perform the pendulum motion while tracing the figure-8 route based on the XY-axes. Such pendulum movement of the rigid body unit 320 looks like a simple pendulum motion and reciprocating motion when viewed from the X-axis direction, and looks like a simple pendulum motion and reciprocating motion when viewed from the Y-axis direction.

In addition, when the pendulum motion is performed based on the XY axes, the length of the holder 400 is fixed so that the height of the holder 400 is also periodically changed.

As a result, due to movement of the power transmission unit 300, the holder 400 and the clothes hanger 800 can be shaken in all directions of the XYZ axes during one revolution of the rotary shaft 210.

In other words, the holder 400 and the clothes hanger 800 may reciprocate not only in the width and height directions of the cabinet 10 but also in the forward and backward directions of the cabinet 10 during one cycle of the pendulum motion.

As a result, the laundry treatment apparatus according to the present disclosure can obtain the effect of removing fine dust from the clothes (the effect of dusting off the clothes) in all directions.

FIG. 12 is a view illustrating a situation in which the clothes hangers 800 do not move integrally with the holder 400.

Referring to FIG. 12, although the power transmission unit 300 and the holder 400 perform an arc-shaped pendulum motion or a figure-8 pendulum motion depending on the direction, the clothes hanger 800 can move independently of the holder 400 due to its own inertial force.

In other words, although the power transmission unit 300 and the holder 400 move in a complex manner, only the hook unit 810 of the clothes hanger 800 is hung on the holder 400, so that the hanger body 820 on which the clothes are hung can move independently of the holder 400.

As a result, the clothes hanger 800 can perform a simple reciprocating motion or pendulum motion in the (+Y)-axis and (-Y)-axis directions, rather than performing a reciprocating motion or a figure-8-shaped pendulum motion while twisting in the XY directions together with the holder 400 due to load of the clothes hanger 800 or load of clothes.

Moreover, when the clothes hanger 800 is lifted upward or downward from the holder 400, the clothes hanger 800 may be twisted at an angle different from that of the holder 400 by inertial force. When loads of the clothes hung on the clothes hangers 800 are different from each other, the twisted angles of the respective clothes hangers 800 may be different from each other.

As a result, the plurality of clothes hangers 800 may collide with each other, or the clothes hung on the plurality of clothes hangers 800 collide with each other, so that the clothes hangers 800 and the clothes may be damaged or worn due to such collision.

In order to address this issue, the laundry treatment apparatus 1 according to the present disclosure may further include the hanger support 430 that enables the clothes hanger 800 to move integrally with the holder 400.

Hereinafter, not only specific embodiments of the power transmission unit 300 and the holder 400, but also specific embodiments of the hanger support 430 applicable to each of the above embodiments will be described with reference to the attached drawings.

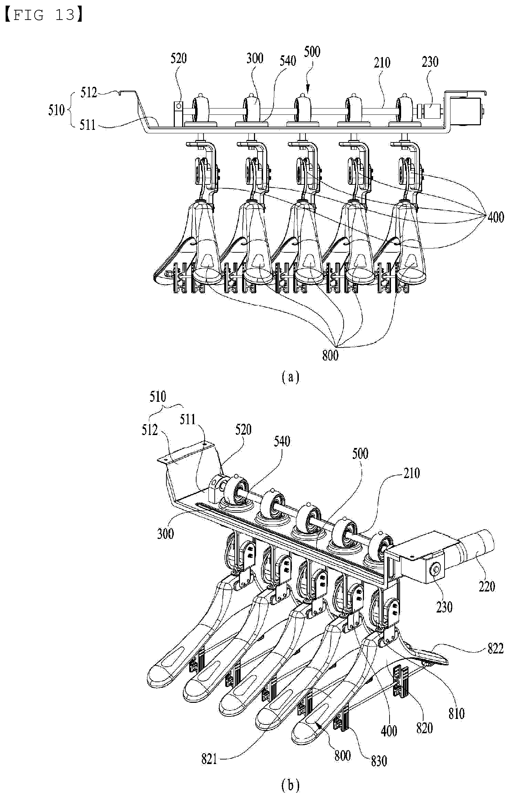

FIG. 13 is a view illustrating one example of the power transmission unit and the holder.

FIG. 13(a) is a front view of the power transmission unit and the holder, and FIG. 13(b) is a perspective view of the power transmission unit and the holder.

In the laundry treatment apparatus according to the present disclosure, the holder 400 may be disposed inside the inner case 20, the drive unit 200 may be disposed outside the inner case 20, and the power transmission unit 300 may interconnect the drive unit 200 and the holder 400 after passing through the inner case 20.

The drive unit 200 may be disposed at a top surface of the inner case 20, and the support unit 500 may be disposed above the inner case 20 to support the drive unit 200.

The power transmission unit 300 passes through a through-hole 25 provided in the inner case 20. One end of the power transmission unit 300 can be coupled to the rotary shaft 210 from the outside of the inner case 20, and the other end of the power transmission unit 300 can be exposed to the inside of the inner case 20 and coupled to the holder 400.

The support 500 may include a coupling body 512 mounted on the frame 12 and a main body 511 extending from the coupling body 512 to support the drive unit 200.

The main body 511 may be disposed parallel to the rotary shaft 210, and the coupling body 512 may be extended to both sides of the main body 511 and may be coupled to the frame 12.

The main body 511 may extend to be bent downward from the coupling body 512, thereby securing a space in which the drive unit 200 is seated.

The main body 511 may be supported in contact with the top surface of the inner case 20, and may be spaced apart from the top surface of the inner case 20 by a predetermined distance.

The rotary shaft 210 may be disposed above the main body 511 while being located between the coupling bodies 512, and the motor 220 can be supported by coupling to the outer surfaces of the coupling bodies 512.

The main body 511 and the coupling body 512 may be formed of a metal plate.

The holder 400 may be disposed below the main body 511, and the power transmission unit 300 may be disposed above the main body 511.

The support 500 provided to penetrate the main body 511 may further include a passage hole 550 through which the power transmission unit 300 can pass, and a support holder 540 for supporting the power transmission unit 300.

The passage hole 550 may be provided to face a through-hole 25, and the power transmission unit 300 and the holder 400 may be arranged to be spaced apart from each other by a predetermined distance in the longitudinal direction of the rotary shaft 210. The support holder 540 may be disposed to correspond to a position where the lower portion of the power transmission unit 300 can be supported.

The power transmission unit 300 may include a connection member configured to pass through the passage hole 550 and the through hole 25, and the holder 400 may be coupled to the connection member at a lower portion of the support 500 and then supported by the support 500.

As a result, the power transmission unit 300 may be provided to connect the holder 400 disposed below the support 500 to the drive unit 200 disposed above the support 500.

As a result, the manufacturer can install the drive unit 200 and the power transmission unit 300 on the support 500, and can fix the support 500 to the upper portion of the inner case 20 so that the power transmission unit 300 can be disposed in the inner case 20. Thereafter, the manufacturer can install the holder 400 in the inner case 20 by coupling the holder 400 to the power transmission unit 300.

As a result, the holder 400 and the power transmission unit 300 can increase the coupling force and the fixing force through the inner case 20 and the support 500.

The clothes hanger 800 may be provided to be hung on the holder 400 so that the clothes can be disposed in the inner case 20.

The drive unit 200 may include a rotary coupling unit 230 that transmits rotational force of the motor 220 to the rotary shaft 210 by coupling the motor 220 to the rotary shaft 210.

The rotary coupling unit 230 may be provided as a gearbox that changes a revolutions per minute (RPM) of the motor 220 and transmits the resultant RPM to the rotary shaft 210, and may be provided as a configuration for connecting the drive shaft of the motor 220 to the rotary shaft 210.

The motor 220 may be disposed at the outer surface of the coupling body 512. The rotary coupling unit 230 may be provided as a gearbox, and may be coupled and fixed to the outer surface of the coupling body 512.

The support 500 may include a shaft coupling unit 520 rotatably supporting one or more of both ends of the rotary shaft 210.

Meanwhile, the clothes hanger 800 may include a hook unit 810 that can be fixed to the holder 400 and a hanger body 820 coupled to the hook unit 810 to hold clothes.

The hanger body 820 may be formed in a triangular shape, and the bottom side of the hanger body 820 may be omitted.

The hook unit 810 may be coupled to an upper end of the hanger body 820.

The clothes hanger 800 may further include a clothes clip (i.e., a clothes tongs) 830 for interconnecting lower ends of the hanger body 820 to grip the clothes.

One surface of the hanger body 820 is supported in contact with the holder 400 so that the hanger body 820 can move integrally with the holder 400.

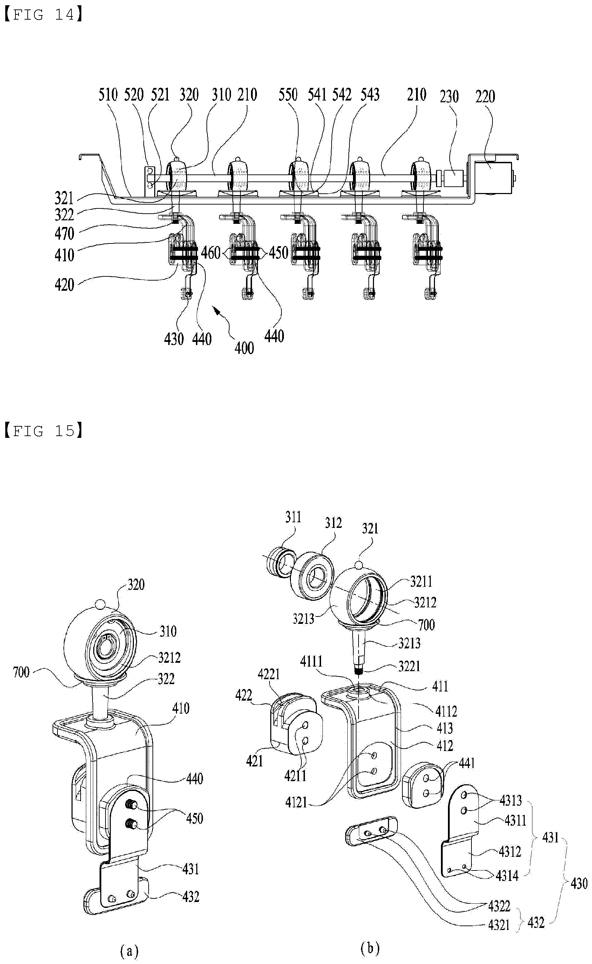

FIG. 14 is a cross-sectional view illustrating a coupling structure between the power transmission unit and the holder shown in FIG. 13.

The rotary shaft 210 can be rotatably fixed through the shaft coupling unit 520 provided at an upper portion of the support plate 510, and the motor 220 may be fixed to the support plate 510 to rotate the rotary shaft 210 through the rotary coupling unit 230.

The power transmission unit 300 may also be implemented as a plurality of power transmission units 300, so that the power transmission units 300 may be spaced apart from each other by a predetermined distance in the longitudinal direction of the rotary shaft 210.

Since the holder 400 is also coupled to and supported by the power transmission unit 300, the holder 400 is also provided as a plurality of holders 400, so that the holders 400 can be spaced apart from each other by a predetermined distance in the longitudinal direction of the rotary shaft 210.

Thus, when the rotary shaft 210 rotates, the plurality of power transmission units 300 move, so that all of the holders 400 can be shaken.

The power transmission unit 300 may include a bearing unit 310 that is obliquely coupled to the rotary shaft 210 and rotates, and a rigid body unit 320 provided to accommodate the bearing unit 310 and provided in a ring shape.

The rigid body unit 320 may include a rigid body 321 provided to receive the bearing unit 310, and a connection bar 322 extending from the rigid body 321 to pass through the support 500 and the inner case 20.

The outer circumferential surface of the rigid body 321 may be provided as a curved surface having a convex outer circumferential surface, and the length of the connection bar 322 may be greater than the thickness or diameter of the connection bar 322.

The connection bar 322 may be provided to pass through a passage hole 550 formed to penetrate the support plate 510.

The holder 400 may include a holding plate 410 coupled to the power transmission unit 300 and extending toward the bottom surface of the inner case 20.

The holding plate 410 may be formed to have a width or length larger than the diameter of the rigid body unit 320. Thus, the holding plate 410 can reliably perform the role of an axis of a pendulum motion shaking clothes.

The holding plate 410 may be coupled to the connection bar 322 and supported by the power transmission unit 300.

The holder 400 may include a hanger unit 420 coupled to the holding plate 410 and provided to allow the clothes hanger 800 or the clothes to be seated thereon.

The hanger unit 420 may be coupled to and fixed to one surface of the holding plate 410 through the first fastening member 460.

The holder 400 may further include a hanger support 430 coupled to the holding plate 410 to support one surface of the hanger.

The hanger support 430 may be coupled to and fixed to the other surface of the holding plate 410 through the second fastening member 450.

The holder 400 may further include an intermediary unit 440 capable of fixing the hanger unit 420 and the hanger support 430 by coupling the hanger unit 420 and the hanger support 430 to the holding plate 410.

The intermediary unit 400 may be disposed between the holding plate 410 and the hanger support 430.

The hanger unit 420 and the intermediary unit 440 may be formed of resin, and each of the hanger unit 420 and the intermediary unit 440 may include a screw hole that can be coupled to the first fastening member 460 and the second fastening member 450.

As a result, although the holding plate 410 is made of a metal material, forming a screw thread in the holding plate 410 can be excluded, thereby simplifying a manufacturing process.

The first fastening member 460 may be inserted from the outer surface of the hanger unit 420 toward the holding plate 410. Also, the first fastening member 460 may be coupled to and fixed to the intermediary unit 440.

The second fastening member 450 may be provided to be inserted from the outer surface of the intermediary unit 440 toward the holding plate 410. In addition, the second fastening member 450 may be coupled to and fixed to the hanger unit 420.

Each of the first fastening member 460 and the second fastening member 450 may be provided in plural, and any one of the first fastening member 460 and the second fastening member 450 may also be omitted as necessary.

Thus, the holder 400 can stably arrange the clothes hanger 800 or the clothes in the inner case 20 through the holding plate 410 and the hanger unit 420, and power received from the power transmission unit 300 can be accurately transmitted to the clothes hanger 800 or the clothes.

The hanger support 430 may extend farther downward than the hanger unit 420 to support one side of the clothes hanger 800, thereby allowing the clothes hanger 800 to move integrally with the holding plate 410.

The support holder 540 may be provided to support a bottom surface of the rigid body 321.

The support holder 540 may include a seating surface 541 for contacting and supporting the bottom surface of the rigid body 321. The support holder 540 may further include a reinforcement surface 543 extending from the support plate 510 that can reinforce the rigidity of the seating surface 541 and distribute the load, and an extension surface 542 through which the reinforcement surface 543 and the seating surface 541 are connected to each other.

Accordingly, when the axial direction of the rigid body 321 rotates in a cone shape around the rotary shaft 210, only the direction of the connection bar 322 is changed and the connection bar 322 also rotates in a cone shape in the changed direction. As a result, each of the holding plate 410 and the hanger unit 420 may move back and forth (i.e., a pendulum motion).

The connection bar 322 is configured such that a region located higher than the support 500 has a longer length than a region located lower than the support 500, so that the amplitude of the holder 400 can be further expanded.

The length of the connection bar 322 may be longer than the diameter of the rigid body 321.

FIG. 15 is a view illustrating an example of the power transmission unit and the holder shown in FIG. 13.

Referring to FIG. 15(a), the rigid body unit 320 may be provided to accommodate the bearing unit 310 and move integrally with the outer circumferential surface of the bearing unit 310. The rigid body unit 320 may be provided with a separation prevention ring 3121 to prevent the bearing unit 310 from being separated in the axial direction.

The connection bar 322 may extend from a lower portion of the rigid body unit 320, and may be coupled to the holding plate 410 after passing through the holding plate 410.

The hanger unit 420 may be coupled to one surface of the holding plate 410, and the intermediary unit 440 or the hanger support 430 may be coupled to the other surface of the holding plate 420.

The fastening member 450 may be provided to couple the hanger unit 420, the intermediary unit 440, and the hanger support 430 to the holding plate 410.

The hanger support 430 may include a support body 431 and a support member 432. The support body 431 may be coupled to the holding plate 420, and may extend farther downward than the hanger unit 420. The support member 432 may be coupled to the support body 431 and may be supported by one side of the clothes hanger 800.

The support body 431 may be provided as a metal plate to guarantee durability and coupling force with the holding plate 410. Even when a load is transferred to the clothes hanger 800, the support body 4321 may prevent deformation of the clothes hanger 800.

The support member 432 may be provided as an elastic member and may be provided to be in surface contact with one surface of the clothes hanger 800. As a result, the support member 432 can increase contact force with the clothes hanger 800, can provide force capable of moving the clothes hanger 800, and can thus prevent damage to the clothes hanger 800.

Referring to FIG. 15(b), the inclined pipe 311 may be provided to accommodate the rotary shaft 210. The inclined pipe 311 may further include a fixing ring 3114 provided at one end thereof and formed to be larger in size than the diameter of the inclined pipe 311. The fixing ring may prevent the rotary bearing 312 from being separated from the inclined pipe 311.

The length of the inclined pipe 311 may be greater than the thickness of the rotary bearing 312. Accordingly, the laundry treatment apparatus can prevent the inclined pipe 311 from being separated from the rotary bearing 312, and can completely transmit the power transmitted from the inclined pipe 311 to the rotary bearing 312.

The rotary bearing 312 may be coupled to the outer circumferential surface of the inclined pipe 311 and fixed to the inclined pipe 311. Accordingly, the shaft of the rotary bearing 312 can move in the same way as the shaft of the inclined pipe 311 moving when the inclined pipe 311 precesses.

The rigid body 321 may be coupled to the outer circumferential surface of the rotary bearing 312 and fixed to the rotary bearing 312.

The rigid body 321 may be provided as a thin circular ring. The rigid body 321 may include a support outer circumferential surface 3213 provided as a spherical shape or an outwardly convex curved surface, an accommodation inner circumferential surface coupled to the outer circumferential surface of the rotary bearing 312, and a support ring 3212 disposed on any one of one surface and the other surface that are opened at the accommodation inner circumferential surface 3211 to prevent separation of the rotary bearing 312.

The thickness or length of the rigid body 321 in the axial direction may be greater than that of the bearing unit 310 in the axial direction. Accordingly, when the bearing unit 310 is accommodated in the rigid body 321, the bearing unit 310 may not protrude outward from the rigid body 321.

The accommodation inner circumferential surface 3211 may be recessed in a shape corresponding to the outer circumferential surface of the bearing unit 310 at the inner circumferential surface of the rigid body 321. The accommodation inner circumferential surface 3211 may form a groove into which the bearing unit 310 is fitted and coupled to the inner circumferential surface of the rigid body 321. Thus, the outer circumferential surface of the bearing unit 310 may be fitted and coupled to the inner circumferential surface of the accommodation inner circumferential surface 3211.

Since the axial thickness of the rigid body 321 is greater than the axial thickness of the bearing unit 310, the accommodation inner circumferential surface 3211 can form a support ring 3212 at both open surfaces of the inner circumferential surface of the rigid body 321.

The support ring 3212 may support both sides of the bearing unit 310 to prevent the bearing unit 310 from being separated from the rigid body 321.

The connection bar 322 may extend downward from the support outer circumferential surface 3213.

The rigid body unit 320 may be formed of a metal material, and may support the holder 400 while shaking the holder 400 with power transmitted from the bearing unit 310.

The diameter of the connection bar 322 may be smaller than the thickness of the rigid body 321 in the axial direction. The connection bar 322 may have a smaller diameter as it extends downward, may be made of a metal material, and may be manufactured integrally with the rigid body 321.

A coupling screw thread 3221 to which a coupling member such as a nut can be coupled may be provided at a lower portion of the connection bar 322.

The connection bar 322 may extend from the rigid body 321, and may serve as a thread or an axis of the pendulum when the rigid body 321 wobbles such that the axis thereof is changed. As a result, when the rigid body 321 is moved by the bearing unit 310, the free end of the connection bar 322 can perform a pendulum motion while tracing an arc shape or a figure-8 shape.

The holder 400 may include a holding plate 410 coupled to and supported by the connection bar 322.

The holding plate 410 is formed in a plate shape made of a metal material and can perform a pendulum motion integrally with the connection bar 322 while supporting the load of the clothes hanger 800.

The holding plate 410 may be formed in a thin plate shape having a thickness smaller than the diameter of the connection bar 322. The holding plate 410 may be coupled to the connection bar 322 while penetrating the connection bar 322, so that the holding plate 410 may extend toward the bottom surface of the inner case 20.

The holding plate 410 may be provided to have a much longer length than the width thereof.

The holding plate 410 may include a first plate 411 through which the connection bar 322 penetrates and is coupled, and a second plate 412 extending from the first plate 411.

The first plate 411 may be disposed obliquely with the connection bar 322 so that the connection bar 322 can pass therethrough. For example, the first plate 411 may be arranged vertically based on the connection bar 322.

The second plate 412 may extend from the first plate 411 toward the bottom surface of the inner case 20. The length of the second plate 412 may be longer than that of the first plate 411.

Accordingly, the amplitude of the clothes hanger 800 may be increased by arranging the hanger unit 420 as far away from the rigid body unit 320 as possible.

The hanger unit 420 may be coupled to one surface of the second plate 412 and may be disposed below the first plate 411. The upper end of the hanger unit 420 may be provided to face the first plate 411.

The hanger unit 420 may be coupled to a lower portion of the second plate 412. The hanger unit 420 may be disposed closer to the lower end of the second plate 412 than to the upper end of the second plate 412.

The hanger unit 420 may include a hanger body 421 coupled to the second plate 412, and a ring groove 4221 in which the hook unit of the clothes hanger 800 can be inserted may be provided at the upper portion of the hanger body 421.

The ring groove 4221 may be recessed to a depth greater than the thickness of the hook unit of the clothes hanger 800 in the hanger body 421. The hanger body 421 may be provided with a ring body 422 spaced apart from the upper portion of the hanger body 421 by a predetermined width corresponding to the hook unit of the clothes hanger 800 while extending from the upper portion of the hanger body 421, and the ring groove 4221 may be provided between the ring body 422 and the upper portion of the hanger body 421.

The hanger body 421 may include a hanger hole 4121 through which the first fastening member 460 passes, and the hanger hole 4121 may be implemented as a plurality of hook holes 4121.

Thus, the hook provided at the upper end of the clothes hanger 800 may be gripped by the ring groove 4221, so that the clothes hanger 800 can be mounted on the hanger unit 420. Thus, the clothes hanger 800 and the clothes hung on the clothes hanger 800 can receive power generated when the holder 410 and the hanger unit 420 perform the pendulum motion.

However, when only the hook provided at the top of the clothes hanger 800 is mounted on the hanger unit 420, there is a possibility that the main body of the clothes hanger 800 performs a pendulum motion again with respect to the holder 410.

As a result, the rigid body unit 320, the holder 410, and the hanger unit 420 can move integrally, but the clothes hanger 800 can move independently.

Therefore, there is a possibility that the clothes hanger 800 does not perform a pendulum motion.

In a situation where a plurality of the holders 410 is provided, if the clothes hung on the clothes hangers 800 mounted on the respective holders 410 are different in length from each other, the amplitude or the reciprocating time of the clothes for each holder 410 is also changed, so that each of the clothes is shaken independently.

In this case, the clothes may collide with each other, so that the clothes cannot move with the intended amplitude, and frictional force occurs between the shaking clothes, thereby causing damage to the clothes.

Therefore, the holder 410 may further include the hanger support 430 to support the main body of the clothes hanger 800, so that the clothes hanger 800 can move is integrally with the holder 410.

That is, the clothes hanger 800 is pushed or supported while the hanger support 430 supports the load of the clothes hanger 800 and clothes, thereby preventing the clothes hanger 800 from moving independently of the holder 410.

As a result, the clothes hanger 800 can perform a pendulum motion together with the holder 410, and the clothes hangers 800 mounted on the plurality of holders 410 may move in the same direction at the same time, thereby maximizing the effect of shaking the clothes.

The hanger support 430 may extend farther downward from the second plate 412 than the hanger unit 420, so that the hanger support 420 can support the clothes hanger 800 mounted on the hanger unit 420.

The hanger support 430 may be provided to contact one surface of the main body of the clothes hanger 800 when the hook of the clothes hanger 800 is mounted on the hanger unit 420.

The hanger support 430 may include a support body 431 in which an upper end is coupled to the second plate 412 and the other end extends until facing one surface of the main body of the clothes hanger 800.

The support body 431 may include a first body 4311 coupled to one surface of the second plate 412, and a first body 4311 extending farther downward from the first body 4311 than the hanger unit 420.

The second body 4312 may be bent toward the hanger unit 420 so that the second body 4312 can be disposed closer to the clothes hanger 800.

The first body 4311 may include a body hole 4313 through which the second fastening member 450 can pass, and a plurality of body holes 4313 may be provided.

The hanger support 430 may include a support member 432 coupled to the support body 431 to support one side of the clothes hanger. The support member 432 may be provided as an elastic member, and may be coupled to and fixed to the second body 4312.

The supporting member 432 is larger in width than the second body 4312, so that one side of the clothes hanger 800 can be supported more reliably.

The second body 4312 may be provided with a second body hole 4314 coupled to the support member 432.

The support member 432 may be provided with a protrusion 4322 inserted into and coupled to the second body hole 4314.

Of course, the support member 432 may be coupled to the second body 4312 through a separate fastening member.

The support member 432 may be provided with a recessed surface that is recessed according to the shape of the second body 4312 in a manner that the support member 432 can be fixed in close contact with the second body 4312. The protrusion 4322 may protrude from the recessed surface.

At least a portion of the first body 4311 may be disposed at a height where the first body 4311 partially overlaps the hanger unit 420. Therefore, the first body 4311 can be coupled to the holding plate 410 together with the hanger unit 420 through one fastening member.

In addition, since each of the support body 431 and the holding plate 410 is provided as a metal plate, it may be difficult to form a screw thread coupled to the fastening member.

Therefore, the intermediary unit 440 may be used, which is coupled to the first fastening member 460 for coupling the hanger unit 420 to the holding plate 410, and is also coupled to the second fastening member 450 for coupling the support body 431 to the holding plate 410.

The intermediary unit 440 may be disposed at a height where the intermediary unit 440 overlaps the hanger unit 420 at the other surface of the holding plate 410.

The second plate 412 may be disposed between the intermediary unit 440 and the hanger unit 420, and the intermediary unit 440 may be disposed between the hanger support 430 and the second plate 412.

The intermediary unit 440 may include an intermediate hole 441. At least one of the first fastening member 460 and the second fastening member 450 may pass through the intermediary unit 440, or may be coupled to the intermediary unit 440.

The intermediate hole 441 may be disposed at a position where the intermediate hole 441 can face the hook hole 4211 and the first body hole 4313.

As a result, the holder 410 may enable the clothes hanger 800 to perform the pendulum motion through the hanger unit 420.

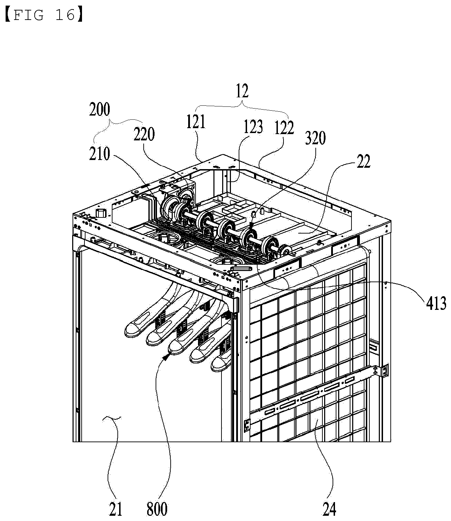

FIG. 16 is a view illustrating another example of the power transmission unit and the holder.

Hereinafter, in order to avoid redundant description, the following description will be given centering upon characteristic portions different from those of the above-described embodiments. Some configurations to be omitted in the following description may be regarded as the same as the previously described configurations.

The inner case 20 may be disposed in a frame of the cabinet 10 formed by the first frame 121, the second frame 122, and the third frame 123, and the drive unit 200 may be provided at an upper portion of the inner case 20.

The power transmission unit 300 may be disposed above the inner case 20. A portion of the holder 410 may be disposed inside the accommodation space 21, and the remaining portions other than the holder portion may be disposed above the inner case 20 by penetrating the upper surface of the inner case 20.

The holder 410 may be directly coupled to the power transmission unit 300 after passing through the inner case 410, and may thus be supported by the power transmission unit 300.

The holder 410 may include a coupling plate 413. One end of the coupling plate 413 may be coupled and fixed to the rigid body 320, and the other end of the coupling plate 413 may be disposed inside the inner case 20.

A support 500 formed of a metal material may be provided between the upper portion of the inner case 20 and a top panel of the cabinet 10.

The support 500 may be supported by the upper surface of the inner case 20, or may be coupled to the frame 12 and supported by the cabinet 10.

Since the support plate 410 of the support 500 is disposed lower than the rotary shaft 210, the coupling plate 412 may pass through the support plate 410.

The motor 220 may be coupled to and supported by the support plate 510.

In addition, the motor 220 may be coupled to and supported by the frame 120, and may be spaced apart from the support plate 510 in any one of the front and rear directions. As a result, the motor 220 can be disposed without restriction of the installation space due to the presence of the support plate 510.

On the other hand, since the rotary shaft 210 is disposed parallel to the support plate 510, a rotary coupling unit for connecting the rotary shaft 210 to the motor 220 may be further provided.

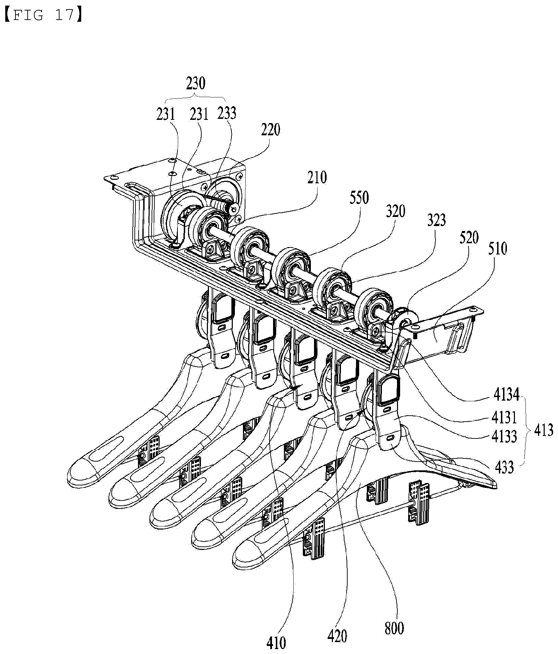

FIG. 17 is a cross-sectional view illustrating a coupling state between the power transmission unit and the holder shown in FIG. 16.

The motor 220 may be disposed such that a drive shaft thereof does not face the support plate 510.

For example, the motor 220 may be disposed behind the support plate 510.

On the other hand, the rotary shaft 210 may be disposed parallel to the support plate 510 at an upper portion of the support plate 510, and may be rotatably supported by a shaft coupling unit 520 provided in the support plate 510.

The rotary coupling unit 230 may be provided to transfer power of the motor 220 to the rotary shaft 210 spaced apart from the motor 220. For example, the rotary coupling unit 230 may include a drive pulley 233 coupled to the drive shaft 210, a rotation pulley 232 provided to rotate together with the rotary shaft 210, and a belt 231 for simultaneously rotating the drive pulley and the rotary pulley by interconnecting the drive pulley and the rotary pulley.

The rotary shaft 210 may be provided with a plurality of power transmission units 300 spaced apart from each other in the longitudinal direction of the rotary shaft 210. The holder 410 may be provided for each power transmission unit 300, so that the holders 410 can be coupled to the power transmission units 300, respectively.

The support plate 510 may be provided with a passage hole 550 through which the coupling plate 413 can pass.

The passage hole 550 may be formed to allow the coupling plate 413 to pass therethrough, and may also have a larger region than a region facing the rigid body unit 320.

Accordingly, when the coupling plate 413 coupled to the rigid body unit 320 performs pendulum motion, the coupling plate may not be restricted by the support plate 510.

The coupling plate 413 may include a coupling end 4131 coupled to the rigid body unit 320, and a plate body 4132 extending from the coupling end toward the inner case 420.

The coupling end 4131 may be coupled to a side surface of the rigid body unit 320. The coupling end 4131 may be provided such that a holding fastening member 4134 can pass therethrough and the holding fastening member 4134 is coupled to the side surface of the rigid body unit 320 to fix the coupling end 4131.

As a result, the connection bar 322 extending from the rigid body unit 320 may be omitted, and the coupling plate 413 can be formed in a straight plate shape, thereby simplifying a configuration, a fabrication process, and a coupling process.

In addition, the coupling plate 413 may be directly coupled to a lower portion of the side surface of the rigid body unit 320, and may more directly receive power of the rotary shaft 210 while moving integrally with the rigid body unit 320, so that the coupling plate 413 can perform pendulum motion.

The clothes hanger 800 may be provided to be mounted on the coupling plate 413.

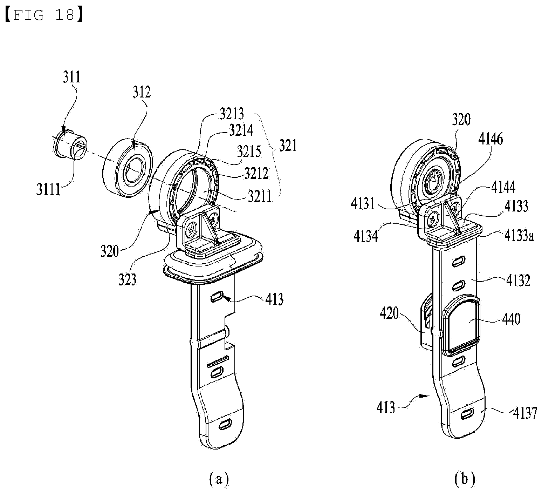

FIG. 18 is a view illustrating an example of a detailed structure of the power transmission unit and the holder shown in FIG. 16.

Referring to FIG. 18(a), the inclined pipe 311 and the bearing unit 310 such as the rotary bearing 312 may be identical in structure to those of the above-described embodiments.

Since the rigid body unit 320 is directly coupled to and supported by the coupling plate 413, the rigid body unit 320 may be provided to have higher rigidity or may be provided to have a greater circumferential surface thickness.

The rigid body 321 may include at least one of reinforcement holes 3214 and reinforcement ribs 3215. Each of the reinforcement holes 3214 may be provided to axially penetrate a space between the inner circumferential surface 3211 accommodating the bearing unit 310 and the outer circumferential surface 3213, or may be recessed in the axial direction. The reinforcement ribs 3215 may be provided to interconnect the inner circumferential surface 3211 and the outer circumferential surface 3213.

In other words, the inner and outer circumferential surfaces of the rigid body 321 may not be arranged to face the outer and inner circumferential surfaces of the plate, but the inner and outer circumferential surfaces 3211 and 3213 may be spaced apart from each other by a certain thickness or more.

As a result, the thickness of the diameter of the rigid body 321 is further increased, so that the rigidity of the rigid body 321 can also be increased.

In addition, as the thickness of the diameter of the rigid body 321 becomes thicker, the area to which the coupling plate 413 can be in contact or fastened can also be enlarged.

Meanwhile, the rigid body 321 may be made of reinforced plastic rather than a metal material.

Accordingly, in order to increase the rigidity of the rigid body 321, the reinforcement holes 3214 may be formed by penetrating the thickness of the rigid body 321 in the axial direction. The plurality of reinforcement holes 3214 may be spaced apart from each other by a predetermined distance along the circumference of the rigid body 21. The reinforcement rib 3215 may be disposed between the reinforcement holes 3214.

As a result, the load transferred to the inner circumferential surface 3211 or the outer circumferential surface 3213 of the rigid body 321 may be distributed to reinforce the rigidity of the rigid body 321.

In addition, since the coupling plate 413 is coupled to the side surface of the rigid body 321, the load transmitted to the side surface of the rigid body 321 is distributed by the reinforcement holes 3214 and the reinforcement ribs 3215, so that the durability of the rigid body 321 can be guaranteed.

In order to further secure a coupling area of the coupling plate 413, the rigid body unit 320 may further include a coupling body 323 protruding outward from the rigid body 321.

The coupling body 323 may be provided to further protrude downward from the outer circumferential surface 3213 of the rigid body 321, and the coupling plate 413 may be coupled and fixed to the rigid body 321 while facing a side surface of a lower portion of the rigid body 321 and a side surface of a lower portion of the coupling body 323.

The coupling body 323 may be provided with a coupling hole to which the holding fastening member 4134 can be coupled.

Meanwhile, the hanger unit 420 may be coupled to one surface of the coupling plate 413.

At this time, the hanger unit 420 may be spaced a predetermined distance from the lower end of the coupling plate 413 toward the upper end of the coupling plate 413, and may be coupled to the coupling plate 413.

A region disposed lower than the hanger unit 420 in the coupling plate 413 may serve as a hanger support 430. That is, the coupling plate 413 may include a support extension unit 433 extending downward from the hanger unit 420.

The support extension unit 433 may be integrally provided with the plate body 4132. In addition, the support extension unit 433 may be disposed to be bent in a direction far away from the hanger unit 420 in the plate body 4132.

Accordingly, when the clothes hanger is coupled to the ring seating groove 4211 provided at the upper end of the hanger unit 420, the support extension unit 433 can be in surface contact with the main body of the clothes hanger so as to support the clothes hanger.

Meanwhile, the coupling plate 413 can be provided with a coupling groove 4135 to which the hanger unit 420 can be coupled. The coupling grooves 4135 may be formed by recessing the side surface of the coupling plate 413 in the width direction. The coupling grooves 4135 may be spaced apart from the lower end of the coupling plate 413 by a predetermined distance.

Accordingly, the hanger unit 420 can be inserted into the coupling groove 4135 in the same manner as in a clip, so that the hanger unit 420 can be coupled to both surfaces of the coupling plate 413.

That is, the hanger unit 420 can be provided integrally with the intermediary unit 440, and a spacing between the hanger unit 420 and the intermediary unit 440 can correspond to the thickness of the plate body 4132.

Accordingly, a plurality of through-holes to be coupled to the fastening member can be omitted.

Referring to FIG. 18(b), the coupling plate 413 may include a coupling end 4131 and a plate body 4132. The coupling end 4131 may be coupled to the coupling body 323 while facing the coupling body 323 of the rigid body 321 at one end thereof. The plate body 4132 may extend downward from the coupling end 4131.

The coupling plate 413 may further include a holder support 4133 for connecting the coupling end 4131 to the plate body 4132.

Since the coupling end 4131 is provided parallel to the side surface of the rigid body 321, the holder support 4133 may extend parallel to the rotary shaft 210 at the lower end of the coupling end 4131.

The holder support 4133 may be provided to be larger than the thickness or width of the plate body 4132 to prevent separation of the sealing unit 700 described later or to be supported by the support holder 540.

The coupling plate 413 may further include reinforcement ribs 416 for connecting the coupling end 4131 to the holder support 4133. Each of the reinforcement ribs 416 may be formed in a triangular shape, so that one surface of the reinforcement rib 416 may extend from the coupling end 4131 and the other surface of the reinforcement rib 416 may extend from the holder support 4133.

The reinforcement rib 416 may be disposed at the center of the width of the coupling end 4131 or the holder support 4133. Alternatively, the plurality of reinforcement ribs 416 may be spaced apart from each other by a predetermined distance in the width direction.

The holding fastening member 4134 may be spaced apart from the reinforcement rib 416 and coupled to the coupling end 4131.

The coupling end 4131 may include an end hole into which the holding fastening member 4134 can be inserted in a region spaced apart from the reinforcement rib 416.

The end hole may be provided in plural and the holding fastening member 4134 may also be provided in plural, so that the end holes and the holding fastening members 4134 can be coupled to the coupling body 323. As a result, the coupling force between the holding plate 410 and the rigid body unit 320 may be further increased.

As described above, among elements of the coupling plate 413, the coupling unit 420 and the extension plate 4137 formed to extend downward from the intermediary unit 400 may serve as the hanger support 430.

That is, at least a portion of the extension plate 4137 may be provided as a support extension unit 433 capable of supporting one surface of the clothes hanger 800.

As a result, the coupling plate 413 may be firmly coupled to the rigid body 321. Thus, as the rigid body 321 moves in the axial direction, the coupling plate 413 can perform pendulum motion in the accommodation space 21.

Meanwhile, the coupling plate 413 may further include a recessed groove 4133a provided along the circumference of the plate body 4132 at a lower portion of the holder support 4133.

The recessed groove 4133a may be provided to correspond to the thickness of the sealing unit 700 to be described later, so that the sealing unit 700 can be fitted and fixed thereto.

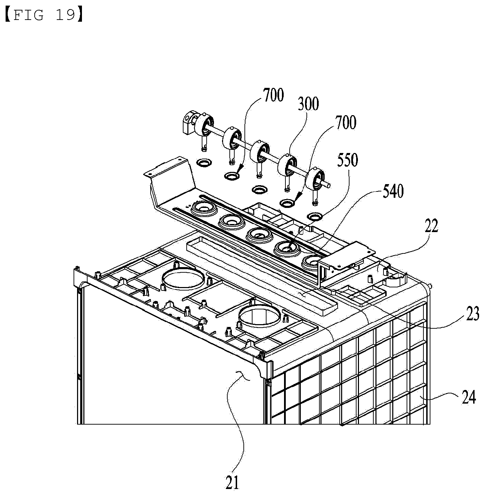

FIG. 19 is a view illustrating an example of the support unit for supporting the power transmission unit or the rotary shaft.

The laundry treatment apparatus 1 according to the present disclosure is configured such that the rotary shaft 210 is disposed outside the inner case 20 and at least a portion of the holder 400 is exposed to the inside of the inner case, thereby supporting the clothes.

Accordingly, the top surface 22 of the inner case 20 should be provided with a through-hole 23 through which at least one of the holder 400 and the power transmission unit 300 can pass.

Meanwhile, the rotary shaft 210 may be rotatably supported by the top surface 23 of the inner case 20.

However, since the rotary shaft 210 is coupled to the power transmission unit 300 and the power transmission unit 300 is provided with the holder 400, the load of the power transmission unit 300 and the load of the holder 400 can be simultaneously added to the rotary shaft 210.

As a result, when the rotary shaft 210 is directly supported by the upper surface 22 of the inner case 20, the upper surface 22 is formed of resin such that there is a possibility that the top surface 22 of the inner case 20 is damaged by excessive load.

In addition, the rotary shaft 210 is disposed in the width direction of the inner case 20 and is provided to rotate, and a plurality of power transmission units 300 is coupled to the rotary shaft 210 so that the loads of the plurality of holders 400 can be supported. Accordingly, when an excessive load is applied between both ends of the rotary shaft 210 and the rotary shaft 210 is curved (or bent), rotation of the rotary shaft 210 is not guaranteed, and the other power transmission unit 300 sags downward or the installation angle of the other power transmission unit 300 is changed, resulting in a damage to the holder 400 having a function of holding the clothes hanger 800 thereon.

Therefore, if the rotary shaft 210 is curved (or bent), there occurs the problem that the laundry treatment apparatus 1 has difficulty not only in shaking the clothes disposed in the inner case 20 but also in holding the clothes inside the inner case 20.

To solve this problem, the laundry treatment apparatus according to the present disclosure may include a support 500.

The support 500 may be provided as a steel plate made of a metal material, so that the support 500 can support all loads to be applied to the rotary shaft 210 and can prevent excessive load from being applied to the rotary shaft 210 or the inner case 20.

The support 500 may be disposed lower than the rotary shaft 210 so as to directly or indirectly support the lower portion of the rotary shaft 210, thereby dispersing the load to be added to the rotary shaft 20. In other words, the support 500 may be provided to support at least one of the rotary shaft 210, the power transmission unit 300, and the holder 400.

In addition, when the support 500 is attached to the upper surface of the inner case 20, it is possible to prevent the upper surface of the inner case 20 from being damaged.

Furthermore, when the support 500 is completely separated or isolated from the upper surface of the inner case 20, any impact or vibration can be prevented from being transmitted to the inner case 20, thereby guaranteeing the durability of the inner case 20.

For example, a portion of the support 500 may be supported by the upper surface of the inner case 20. However, the support 500 may be coupled to the frame 12 to support most loads through the cabinet 10.

The support 500 may include a support plate 510, at least a portion of which is disposed under the rotary shaft 210. The support plate 510 may be provided with a steel plate made of metal.

The support plate 510 may be disposed between the cabinet 10 and the top surface 22 of the inner case.

The support plate 510 is disposed below the rotary shaft 210 to shield the rotary shaft 210 even when the rotary shaft 210 is disposed in the inner case 210 or exposed to the inside of the inner case 210. Therefore, it is possible to prevent the user from hanging on or touching the rotary shaft 210.

In addition, at least a portion of the support plate 510 may be disposed below the rotary shaft 210. Accordingly, the support plate 510 can directly or indirectly support the lower portion of the rotary shaft 210, thereby preventing the rotary shaft 210 from being bent.

The support 500 may include a shaft coupling unit 520 rotatably supporting the rotary shaft 210. The shaft coupling unit 520 may be coupled to the upper portion of the support plate 510 to support both ends of the rotary shaft 210.

Meanwhile, the support 500 may have one or more passage holes 550 formed to pass through the support plate 510 such that at least a portion of the power transmission unit 300 or the holder 400 can pass through the passage holes 550. The passage holes 550 may be disposed to correspond to the numbers and positions of the power transmission units 300 or the holders 400. The passage holes 550 may be arranged in the longitudinal direction of the rotary shaft 210. The passage holes 550 may be disposed in a region corresponding to the through-hole 23.

Although it is shown that the through hole 23 is penetrated to correspond to the shape of the support plate 510 for convenience of description, the scope or spirit of the present disclosure is not limited thereto, and the through hole 23 can be provided as the passage hole 550 or can also be provided as a plurality of holes each having a larger diameter than the passage hole 550 as necessary.

The support 500 may further include a support holder 540 that is provided at the support plate 510 to support the power transmission unit 300 or the holder 400.

The support holder 540 may be provided to support the lower portion of the power transmission unit 300, and may be recessed downward from the support plate 510 in a manner that the power transmission unit 300 can perform the reciprocating motion or the wobble motion. The support holder 540 may be formed to protrude from the support plate 510 to reinforce the rigidity of the support plate 510 and to distribute a load applied to the support holder 540.

Thus, when a user hangs on the rotary shaft 210 or the clothes hanger 800, or when an excessive load is applied only to a specific holder 400, the support holder 540 supports the power transmission unit 300 to distribute such excessive load to the support plate 510, thereby preventing deformation of the rotary shaft 210 or preventing damage to the power transmission unit 300 or the holder 400.

The support holder 540 may have a passage hole 550 therein.

The laundry treatment apparatus according to the present disclosure may further include a sealing unit 700 for shielding the passage hole 500 to prevent hot air or stream from leaking into the passage hole 550.

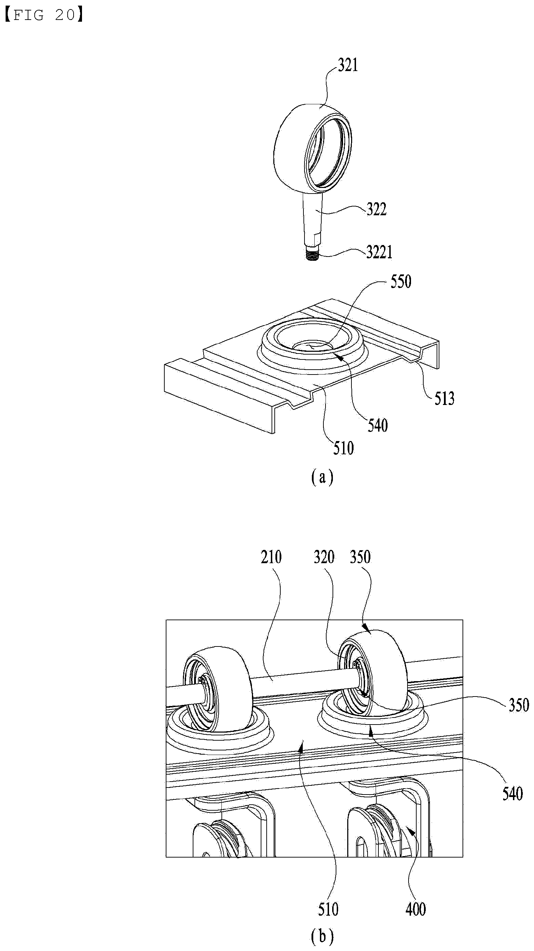

FIG. 20 is a view illustrating an example of the support holder 540.

Referring to FIG. 20(a), the support holder 540 may be provided such that a region facing the rigid body unit 320 is recessed downward.

Therefore, the support holder 540 can accommodate and support at least a portion of the rigid body unit 320. The support holder 540 may support the load applied to the rotary shaft 210 and all of the power transmission units 300 while supporting the rigid body unit 320. Moreover, the support holder 540 can distribute a load applied to the support holder 540 in various directions according to a recessed shape, thereby preventing the load from being concentrated in a specific area and direction.

In addition, the support holder 540 may protrude upward from the support plate 510. Therefore, even if the support plate 510 is spaced apart from the rigid body unit 320, the support holder 540 may contact and support the lower portion of the rigid body unit 320.

In addition, as the support holder 540 protrudes upward from the support plate 510, the rigidity of the support plate 510 can be greatly increased. In addition, the load applied to the support holder 540 can be distributed in more various directions, and a protruding portion can also apply elastic force like a cantilever, so that the rigidity and durability of the support holder 540 can be greatly increased.

Although the connection bar 322 is extended to the rigid body unit 320, the connection bar 322 can pass through the passage hole 550 provided in the support holder 540, and only the lower portion of the rigid body unit 320 can be supported by the support holder 540.

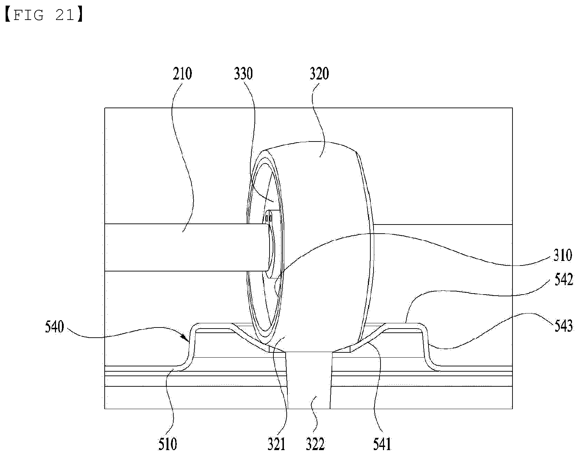

Referring to FIG. 21(b), the support holders 540 may be disposed below the plurality of rigid body units 320, respectively.

The support holder 540 may be provided to prevent contact with the rotary shaft 210, and may be provided to partially accommodate a lower region of the rigid body unit 320.

The support holder 540 may also be provided such that the rigid body unit 320 can be in surface-contact or line-contact with the lower region of the rigid body unit 320.

In addition, the support holder 540 may be spaced apart from the rigid body unit 320 by a predetermined distance so as to come into contact with the lower portion of the rigid body unit 320 when an excessive load is applied to the rigid body unit 320.

Thus, the support holder 540 may prevent the rigid body unit 320 from descending by a certain distance or less, thereby preventing permanent deformation of the rotary shaft 210 from occurring.

Meanwhile, the top surface of the support holder 540 accommodating and supporting the rigid body unit 320 may form a partial spherical surface. Accordingly, the axial direction of the rigid body unit 320 can be changed and at the same time the rigid body unit 320 can perform the reciprocating motion or the pendulum motion.

FIG. 21 is a view illustrating a specific shape of the support holder 540.

The support holder 540 may indirectly support the rotary shaft 210 by supporting the power transmission unit 300.

When the bearing unit 310 precesses with respect to the rotary shaft 210, the rigid body unit 320 can also rotate in the axial direction with respect to the rotary shaft 210.

Accordingly, the bottom surface of the rigid body unit 320 may rotate while tracing a circular arc shape.

Accordingly, the support holder 540 may include a seating surface 541 that is recessed downward to support the rigid body unit 320 while allowing movement of the rigid body unit 320.

The radius of curvature of the seating surface 541 may be equal to or larger than the radius of curvature of the outer circumferential surface of the rigid body unit 320. Accordingly, even if the seating surface 541 contacts the rigid body unit 320, the movement of the rigid body unit 320 can be allowed.

The width of the seating surface 541 may be greater than the axial thickness of the rigid body unit 320. A width of the seating surface 541 may be larger than an axial thickness of the outer circumferential surface 3213 of the rigid body unit 320. Thus, the seating surface 541 can support the entirety of the bottom surface of the rigid body unit 320.

Meanwhile, the depth of the seating surface 541 is smaller than the width of the seating surface 541 so as not to interfere with the movement of the rigid body unit 320.

The seating surface 541 may be formed in a spherical shape, and may have a radius of curvature corresponding to a part of the sphere.

That is, the seating surface 541 may be provided in a funnel shape that is recessed toward the passage hole 550 therein.

The radius of curvature of the seating surface 541 may be greater than or equal to the radius of curvature of a trajectory along which the outer circumferential surface of the rigid body unit 320 moves.

The radius of curvature of the seating surface 541 may be greater than or equal to the radius of curvature of the outer circumferential surface of the rigid body unit 320.

As a result, when the rigid body unit 320 moves while tracing a circular shape or a spherical shape with respect to the rotary shaft 210, the seating surface 541 can support the rigid body unit 320 in surface contact, so that the load of the rigid body unit 320 can be distributed in a wider variety of directions within a wider region.

The seating surface 541 may be bent downward from the support plate 510.

Meanwhile, the support holder 540 may further include a reinforcement surface 543 capable of lifting the seating surface 541 from the support plate 510 toward the rigid body unit 320. The reinforcement surface 543 may protrude upward from the support plate 510, and the seating surface 541 may be provided inside the reinforcement surface 543.

The reinforcement surface 543 may protrude from the support plate 510 to reinforce the rigidity of the support plate 510, and the load applied to the seating surface 541 can be distributed in the vertical direction from the support plate 510.

In addition, the support holder 540 may further include an extension surface 542 extending from the inner circumferential surface of the reinforcement surface 543 toward the seating surface 541.

That is, the extension surface 542 may be provided in the reinforcement surface 543, the seating surface 541 may be provided in the extension surface 542, and the passage hole 550 may be provided in the seating surface 541.

The extension surface 542 may include the seating surface 541 provided on an inner circumferential surface thereof, and may include the reinforcement surface 543 provided on an outer circumferential surface thereof.

The extension surface 542 may be disposed parallel to the support plate 510 or may be formed in an upwardly convex shape.

Thus, the extension surface 542 radially distributes the load received from the seating surface 541 in a radial direction parallel to the support plate 510, thereby preventing deformation of the seating surface 541.

Meanwhile, the reinforcement surface 543 may protrude upward such that the lower portion of the seating surface 541 can be prevented from protruding downward from the support plate 510. In other words, the height of a protrusion of the reinforcement surface 543 may be greater than the depth of a depression of the seating surface 541.

As a result, the seating surface 541 can be prevented from protruding downward from the support plate 510, thereby preventing a user from being injured by the seating surface 541.

The passage hole 550 may be provided at the inner circumferential surface of the seating surface 541, and the connection bar 322 may pass through the passage hole 550.

The support holder 540 can support the rigid body 320 while distributing the load transmitted to the rigid body unit 310 in multiple directions, because the support holder 540 includes a spherical seating surface 541, a ringshaped extension surface 542, and a vertical or inclined reinforcement surface 543.

Furthermore, the extension surface 542 and the reinforcement surface 543 may provide greater elastic force to the seating surface 541 like a cantilever, so that durability of the entire support holder 540 can be guaranteed.

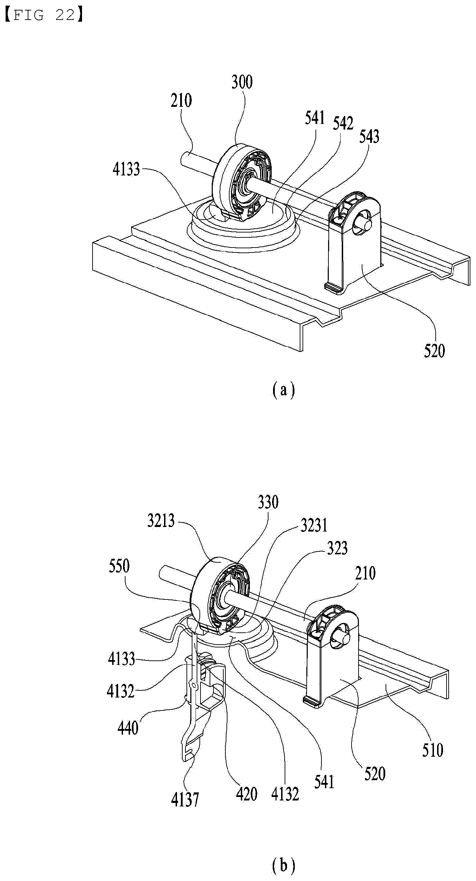

FIG. 22 is a view illustrating another example of the support 500.

Referring to FIG. 22(a), the support 500 may be provided to directly support the holder 400. The support 500 may indirectly support the power transmission unit 300 or the rotary shaft 210 while supporting the holder 400.

The support 500 may be provided to support the holder 400 passing through the support plate 510.

The seating surface 541 provided on the support holder 540 may be provided to support the holder 400.

Specifically, the seating surface 541 may be provided to support the coupling plate 413 directly coupled to the rigid body unit 320.

The coupling plate 413 may include a holder support 4133 that protrudes from the coupling end 4131 much larger than the diameter of the passage hole 550 and is seated on the seating surface 541.

Whereas the coupling plate 413 is provided to penetrate the passage hole 550 provided in the seating surface 541, the coupling plate 413 can be supported by the top surface of the seating surface 541 through the holder support 4133.

Since the seating surface 541 has a circular shape when viewed from the top, a central portion of the seating surface 541 has the largest diameter.

This is because the diameter of the rigid body unit 320 is larger than the width of the coupling plate 413, so that it is most preferable that the area corresponding to the rigid body unit 320 be centered on the seating surface 541.

Therefore, since the rigid body unit 320 is located at the center of the seating surface 541 and the coupling plate 413 is coupled to and fixed to the side surface of the rigid body unit 320, the passage hole 550 can also be formed to be biased from the center of the seating surface 541 to one side of the seating surface 541 in correspondence with the position of the coupling plate 413.

The seating surface 541 may be provided as a curved surface such that the axial direction of the seating surface 541 can rotate with respect to the rotary shaft in the same manner as in precession of the rigid body unit 320.

Therefore, the holder support 4133 may also be provided to have a curved bottom surface such that the holder support 4133 can be supported by the seating surface 541 while being in surface contact with the seating surface 541.

Meanwhile, the plate body 412 may be provided in a bar shape rather than a plate shape in order not to excessively increase the diameter of the passage hole 550.

Specifically, the plate body 4132 may further include a first plate body 4132a provided in a rod shape extending from the holder support 4133 to a region to which the hanger unit 420 is coupled. The first plate body 4132a may have a much smaller width than the main body of the plate body 4132 to which the hanger unit 420 is coupled.

As a result, the plate body 4132 passes through the passage hole 550, and can stably support the hanger unit 420 through the main body disposed under the first plate body 4132a without being disturbed by the seating surface 541 during pendulum movement thereof.

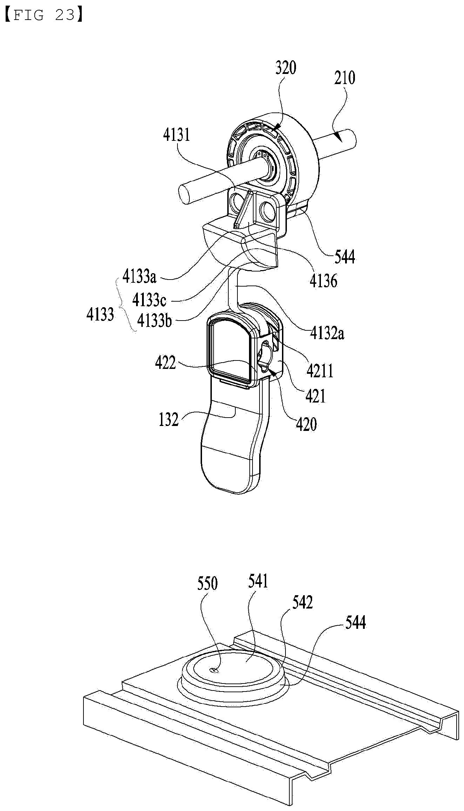

FIG. 23 is a view illustrating a detailed structure of the coupling plate 413 applied to the structure of FIG. 22.

The holder support 4133 may include a holder body 4133a formed to be thicker than the coupling end 4131, and a body surface 4133b that is provided below the holder body 4133a and has a curved surface supported by the seating surface 4133b.

The holder body 4133a may extend in a direction parallel to the rotary shaft 210 from the coupling end 4131.

The body surface 4133b may have a smaller area than the seating surface 541, and the seating surface 541 may have a radius of curvature equal to or greater than that of the body surface 4133b.

The holder support 4133 may further include an extension surface 4133c extending downward from the coupling end 4131 to secure the thickness of the body surface 4133b.

The body surface 4133b may be provided to extend the holder body 4133a and the extension surface 4133c.

The hanger unit 420 may be coupled to and fixed to a lower portion of the first plate body 4132a among the plate bodies 4132.

The hanger unit 420 may include a first hanger body 421 supported by one surface of the plate body 4132, and a second hanger body 422 supported by the other surface of the plate body 4132.

Each of the first hanger body 421 and the second hanger body 422 may have a larger width than the plate body 4132, and the first hanger body 421 and the second hanger body 421. The first hanger body 421 and the second hanger body 422 may be spaced apart from each other by a thickness of the plate body 4132, and one surface of the first hanger body 421 and one surface of the second hanger body 422 may be formed to extend so that the first hanger body 421 and the second hanger body 422 can be formed integrally.

Thus, it can be seen that any one of the first hanger body 421 and the second hanger body 422 serves as the intermediary unit 440. The first hanger body 421 may be provided with a ring coupling groove 4211 in which the hook of the clothes hanger can be seated.

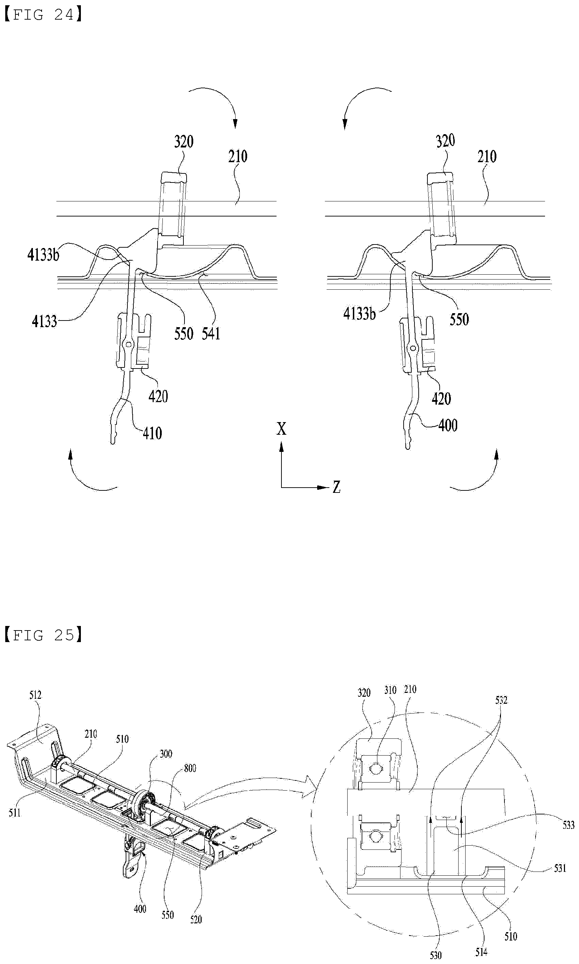

FIG. 24 is a view illustrating an example of the coupling plate 413 performing a pendulum motion.

Referring to FIG. 24(a), the rigid body unit 320 is coupled to the rotary shaft 210, and the coupling end 4131 is coupled to the lower portion of the rigid body unit 320.

The holder support 4133 may extend from the coupling end 4131 and may be seated on the seating surface 541 around the passage hole 550, so that the holder support 4133 can be supported by the seating surface 541.

The rigid body unit 320 may be arranged to be inclined so as to face upward with respect to the rotary shaft 210. The holding plate 410 may be arranged to be inclined to the left in response to the degree of inclination of one surface of the rigid body unit 320 coupled to the coupling end 4131.

Referring to FIG. 24(b), the rotary shaft 210 rotates so that the rigid body unit 320 may be disposed inclined to face downward with respect to the rotary shaft 210. The holder support 4133 is supported by the seating surface 541 and can rotate based on the passage hole 550. The holding plate 410 can be arranged to be inclined to the right in response to the degree of inclination of one surface of the rigid body unit 320 coupled to the coupling end 4131.

Thus, the holding plate 410 may be provided to perform pendulum motion.

The support holder 540 may support the holding plate 410, thereby distributing loads applied to the power transmission unit 300 and the rotary shaft 210.

In addition, the support holder 540 may indirectly support the power transmission unit 300 and the rotary shaft 210.

FIG. 25 is a view illustrating an example of the support 500 directly supporting the rotary shaft 210.

The support 500 may include a support pillar 530 which surrounds the rotary shaft 210 or is in contact with the lower portion of the rotary shaft 210, thereby supporting the rotary shaft 210.

The support pillar 530 may be provided to support an intermediate region of the rotary shaft 210, so that the support pillar 530 can prevent the rotary shaft 210 from being bent downward. Here, the intermediate region is disposed between both ends of the rotary shaft 210.

The support pillar 530 may be provided to contact and support the bottom surface of the rotary shaft 210. In a situation where the support pillar 530 is spaced apart from the bottom surface of the rotary shaft 210 by a predetermined distance, when force applied to the support pillar 530 reaches a region where the rotary shaft 210 is curved to an elastic threshold or greater, the rotary shaft 210 can be supported by the support pillar 530.

Although not shown in the drawings, the support pillar 530 may also be provided as a structure that completely rotatably surrounds the rotary shaft 210 and at the same time supports the rotary shaft 210.

The support pillar 530 may include a pillar body 531 coupled to the support plate 510, and pillar grooves 532 provided at an upper portion of the pillar body 531 to support the rotary shaft.

The pillar groove 532 is provided as a groove in which the upper end of the pillar body 531 is recessed to accommodate the rotary shaft 210, and the upper portion of the pillar groove 532 is opened so that the rotary shaft 210 can be detachably coupled to the pillar groove 532.

Although not shown, a bearing configured to rotatably support the rotary shaft 210 may also be provided at the inner circumferential surface of the pillar groove 532.

Alternatively, the support pillar 530 may also be implemented as a plurality of support pillars that is spaced apart from each other by a predetermined distance in the longitudinal direction of the rotary shaft 210. That is, the plurality of support pillars 530 may be provided to support the remaining regions other than both ends of the rotary shaft 210.

The support pillars 530 may be disposed in the passage hole 550 or the through hole 23 while being spaced apart from each other.

The inner circumferential surface of the pillar groove 532 may include a support protrusion 5321 protruding from the inner circumferential surface of the pillar groove 532 to support the rotary shaft 210.

The support protrusion 5321 may be provided along the inner circumferential surface of the pillar groove 532.

The support protrusion 5321 may be provided as a plurality of support protrusions 5321 so that the support protrusions 5321 are spaced apart from each other by a predetermined distance in the longitudinal direction of the rotary shaft 210.

The support pillar 530 can minimize frictional force by minimizing the size of a contact region with the rotary shaft 210 through the support protrusion 5321, and when the rotary shaft 210 is bent downward, the support pillar 530 can intensively provide strong reaction force to the rotary shaft 210.

As a result, the support 500 may support both ends of the rotary shaft 210 through the shaft coupling unit 520, and may support a region between both ends of the rotary shaft 210 through the support pillar 530.

The support pillar 530 may be disposed between the shaft coupling units 520.

As a result, the support 500 may be in contact with the rotary shaft 210 to directly support the rotary shaft 210.

In addition, as described above, the support 500 can indirectly support the rotary shaft 210 while supporting the power transmission unit 300 coupled to the rotary shaft 210 or the holder 400 coupled to the power transmission unit 300.

The support 500 may include a support plate 510 disposed parallel to the rotary shaft 210 at a lower position than the rotary shaft 210.

Specifically, in the support plate 510, only a portion facing the lower portion of the rotary shaft 210 may be disposed at a lower position than the rotary shaft 210, and the remaining regions other than both ends of the rotary shaft 210 may be disposed at a higher position than the rotary shaft 210.

The entirety of the support plate 510 is made of a metal material, so that strong rigidity and certain elasticity can be guaranteed. Accordingly, the support plate 510 can support the load of at least one of the drive unit 200, the holder 400, and the power transmission unit 300.

The support plate 510 may include a main body 511 disposed below the rotary shaft 210, and a coupling body 512 extending from both sides of the main body 511 to support the main body 511.

The coupling body 521 may include an inclined body 5122 extending upward from both ends of the main body 511 to form a space in which the rotary shaft 210 is disposed, and a fixing body 5121 extending from the inclined body 5122 in a direction farther away from the rotary shaft 210 so that the fixing body 5121 can be seated on the frame 12.

The fixing body 5121 may be provided with a fixing hole 5123 that can be fixed to the frame 12 with bolts or the like.

The support 500 may include a shaft coupling unit 520 coupled to the main body 511 to rotatably support both ends of the rotary shaft 210. The shaft coupling unit 520 may include a bearing that protrudes upward from the main body 511 and is rotatably coupled to at least one of both ends of the rotary shaft 210.

The main body 511 may support the load of the rotary shaft 210 through the shaft coupling unit 520.

The main body 511 may contact the top surface of the inner case 20 to be supported by the inner case 20.

Alternatively, the main body 511 may be spaced apart from the top surface of the inner case 20, and may be supported by the frame 12 forming the frame of the cabinet 10 through the coupling body 521.

As a result, it can be seen that the support 500 is supported by at least one of the cabinet 10 and the inner case 20.

The main body 511 may support the rotary shaft 210, the power transmission unit 300 may be coupled to the rotary shaft 210, and the power transmission unit 300 may be provided with a holder 400, thereby supporting all loads of the rotary shaft 210, the power transmission unit 300, and the holder 400.

Therefore, the support plate 510 may further include a reinforcement bead 513 provided to be bent along the extension direction of the main body 511 so that the rigidity of the main body 511 can be guaranteed.

On the other hand, since the main body 511 of the support plate 510 is disposed below the rotary shaft 210, the support plate 510 may be provided with a passage hole 550 through which at least one of the power transmission unit 300 coupled to the rotary shaft 210 and the holder 400 coupled to the power transmission unit 300 can pass.

Thus, although the rotary shaft 210 is disposed above the support plate 510 and at least a portion of the support portion 400 is disposed below the support plate 510, the support portion 400 or the power transmission unit 300 can be prevented from interfering with the support plate 510.

Meanwhile, the support 500 may further include a support pillar 530 capable of supporting a region between both ends of the rotary shaft 210 by contacting the rotary shaft 210.

Although an excessive load is applied to the rotary shaft 210 due to the presence of the support 500, bending of the rotary shaft 210 can be prevented. Therefore, the rotary shaft 210 is prevented from being bent so that the movement of the rigid body unit 320 and the bearing unit 310, each of which traces a circular shape in the axial direction, can be maintained, so that the function of shaking the clothes by the laundry treatment apparatus can always be guaranteed.

In addition, since the support 500 is disposed at least partially lower than the rotary shaft 210, the rotary shaft 210 can be prevented from being exposed to the user, so that accidents that can be generated when the user hangs on the rotary shaft 210 or touches the rotary shaft 210 can be prevented from occurring.

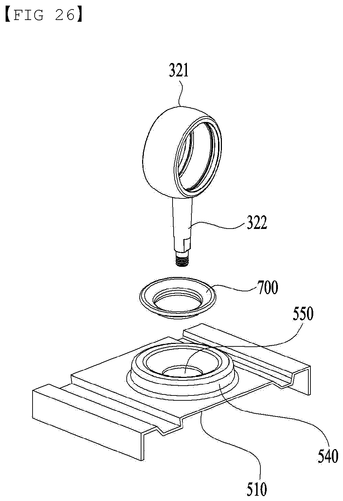

FIG. 26 is a view illustrating an example of a sealing unit of the laundry treatment apparatus according to the present disclosure.

The laundry treatment apparatus according to the present disclosure may include a sealing unit 700 for preventing hot air or steam from leaking into the through hole 23.

The through hole 23 may be shielded by the support frame 510, and the passage hole 550 may be provided to face the through hole 23. Since the passage hole 550 must allow the pendulum motion of the power transmission unit 300 or the holder 400, the through hole 550 has a larger diameter than each of the power transmission unit 300 and the holder 400 formed to penetrate the through hole 330.

For example, the passage hole 550 has a larger diameter than the connection bar 322 or the coupling plate 413.

Therefore, there is a possibility that hot air or steam is discharged outside through the passage hole 550.

The sealing unit 700 may be provided to prevent hot air or steam from leaking into the passage hole 550.

The sealing unit 700 may be seated on the support frame 510 to shield the passage hole 550.

The sealing unit 700 may surround the power transmission unit 300 or the holder 400 within the passage hole 550, and at the same time may prevent hot air or stream from leaking into a spacing between the passage hole 550 and the power transmission unit 300 or between the passage hole 550 and the holder 400.

The sealing unit 700 may include a through hole 720 through which the holder 400 or the power transmission unit 300 passes, and a sealing body 710 extending from the transmission hole 720 to seal the passage hole 550.

The sealing body 710 may be provided to allow movement of the holder 400 or the power transmission unit 300.

The sealing body 710 may be seated on the support frame 510 to seal the passage hole 550.

On the other hand, the sealing unit 700 may further include a fixing unit 730 for preventing prevents the sealing unit 700 from being separated or isolated from the passage hole 550.

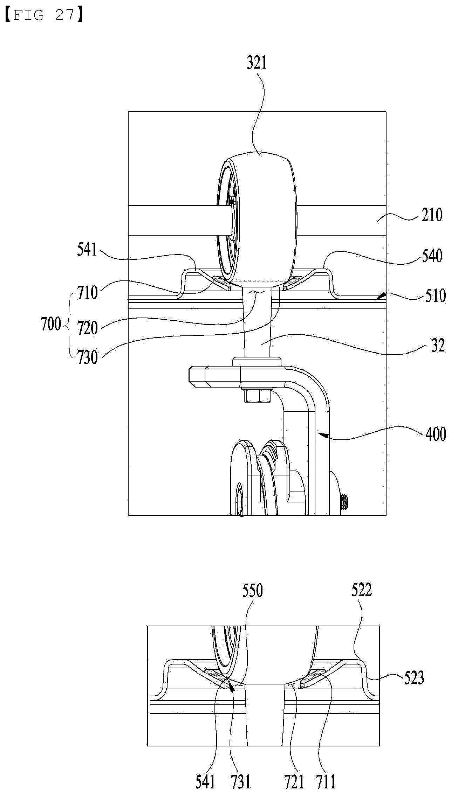

FIG. 27 shows one example of the sealing unit.

Specifically, the sealing unit 700 may include a first sealing unit 701 seated on the support holder 540 to block steam or hot air from leaking into the passage hole 550.

The first sealing unit 701 may include a first sealing body 711 seated on the support holder 540 to support the lower portion of the holder 400 or the power transmission unit 300.

The first sealing body 711 may be seated on the support holder 540 to support a lower portion of the holder 400 or the power transmission unit 300.

The first sealing body 711 may be provided in the shape of a circular ring plate accommodated in the seating surface 541 of the support holder 540.

The bottom surface of the first sealing body 711 is seated on the seating surface 541, and the top surface of the first sealing body 711 is provided to support a part of the holder 400 or the lower portion of the power transmission unit 300.

The first sealing unit 701 may include a first transmission hole 721 provided to penetrate the first sealing body 711 so as to communicate with the passage hole 550.

The first sealing body 711 may have a shape corresponding to the top surface of the seating surface 541. The first sealing body 711 may be provided with a spherical or curved surface in which each of the top surface and the bottom surface is provided as a downwardly convex spherical surface or a downwardly curved surface.

The seating surface 541 may be provided in a funnel shape having a larger width than the depth thereof, and the first sealing body 710 may also be provided in a funnel shape having a larger width than the depth thereof. In other words, the seating surface 541 is provided in a funnel shape that has a smaller diameter in a downward direction. The first sealing body 710 may be provided in a funnel shape that has a smaller diameter in a downward direction.

The seating surface 541 may be provided with a smaller diameter or width in the direction from the outer circumferential surface to the passage hole 550. The first sealing body 710 may be provided with a smaller diameter or width in the direction from the outer circumferential surface to the first transmission hole 721.

As a result, at least a portion of the bottom surface of the first sealing body 711 may be in surface contact with the seating surface, so that the first sealing body 711 can be supported.

Thus, the first sealing body 711 may be seated on the seating surface 541, and can be prevented from escaping to the outside of the support holder 540.

The diameter or width of the first sealing body 711 may be larger than the axial thickness or width of the bottom surface of the holder 400 or the power transmission unit 300.

As a result, the first sealing body 711 can continuously support the bottom surface of the holder 400 or the power transmission unit 300 during the process of moving the holder 400 or the power transmission unit 300 with respect to the rotary shaft 210.

For example, the first sealing body 711 may be made of a metal material, and at least one of hardness, strength, and specific gravity may be greater than that of the support holder 540.

The first sealing body 711 may be seated on the seating surface 541 to support the rigid body unit 320. The first sealing body 711 may be provided to shield the passage hole 550 while supporting the bottom surface of the rigid body unit 320.

When the connection bar 322 is provided in the rigid body unit 320, the connection bar 322 can penetrate the first sealing body 711 through the first transmission hole 721 provided at the inner circumferential surface of the first sealing body 711.

The first transmission hole 721 may have a larger diameter than the connection bar 322. Thus, the above-described configuration can not only allow the connection bar 322 to penetrate the first transmission hole 721, but also can prevent the connection bar 322 from being disturbed by the first transmission hole 721 even when the connection bar 322 performs the pendulum motion.

The diameter of the first transmission hole 721 may be smaller than the axial thickness of the rigid body unit 320. As a result, the bottom surface of the rigid body unit 320 may be provided to shield the first transmission hole 721.

The rigid body unit 320 may perform pendulum motion at an upper portion of the first transmission hole 721 with respect to the first transmission hole 721. Even in this case, the bottom surface of the rigid body unit 320 may always be provided to shield the first transmission hole 721.

As a result, the first sealing body 711 may be provided to shield the passage hole 550, and the rigid body unit 320 may be provided to shield the first transmission hole 721 formed at the inner circumferential surface of the first sealing body 711. Therefore, hot air or steam can be prevented from leaking into the passage hole 550 and the first transmission hole 721.

On the other hand, the first sealing unit 700 may include a first fixing unit 731 that can prevent the first sealing body 710 from being separated from the support holder 540 or can prevent the position of the first sealing body 710 from being changed.

The first fixing unit 731 may be formed in a protrusion ring shape that protrudes from the first sealing body 711 or the outer circumferential surface of the first transmission hole 721 so as to be at least partially inserted into the passage hole 550.

The diameter of the first fixing unit 731 may be equal to or smaller than the diameter of the passage hole 550. Thus, the first fixing unit 731 may be disposed inside the passage hole 550.

The first fixing unit 731 may extend downward from the bottom surface of the first sealing body 711 or the inner circumferential surface of the first transmission hole 721.

The first fixing unit 731 may also be provided in a hook shape, a cross section of which can be supported by the inner circumferential surface of the passage hole 550.

Thus, even if the first sealing body 711 is pressed by the movement of the rigid body unit 720 or vibration generated by the rotary shaft 210 or clothing is transmitted, the first fixing unit 731 is supported by the inner circumferential surface of the passage hole 550 to prevent the first sealing body 711 from being separated from or slipping on the seating surface 541.

As a result, it is possible to prevent the arrangement position of the first sealing unit 701 from being changed due to the first fixing unit 731.

The first fixing unit 731 may be provided so that the bottom surface thereof is not exposed to the lower portion of the seating surface 541 or the lower portion of the support plate 510. A protruding length of the first fixing unit 731 may be smaller than a vertical thickness of the passage hole 550.

Accordingly, it is possible to prevent the first fixing unit 731 from coming into contact with the user's body or constituent components located inside the inner case 20.

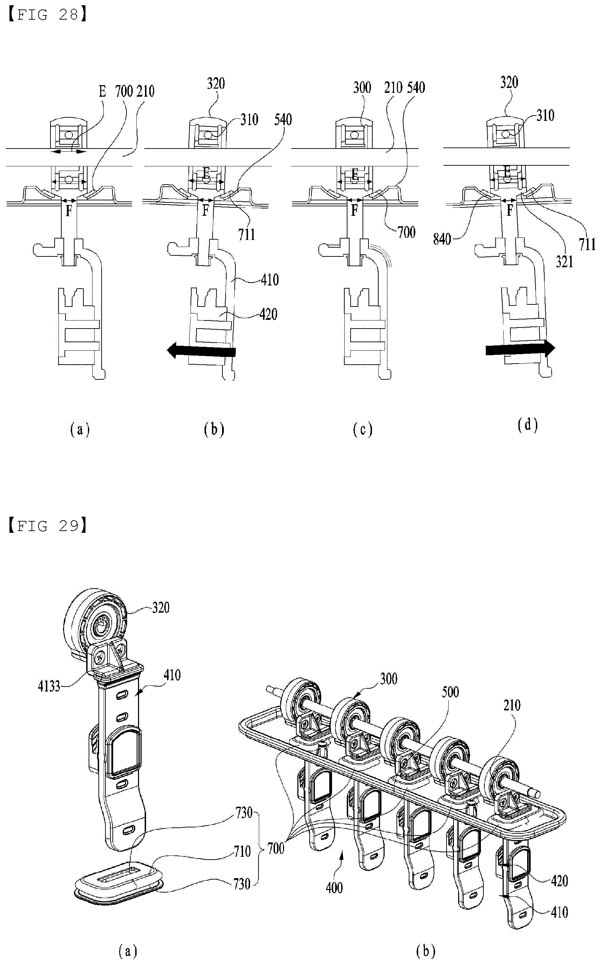

FIG. 28 is a view illustrating operation methods of the first sealing unit 701 according to the present disclosure.

Referring to FIG. 28(a), the width of the upper portion of the seating surface 541 may be greater than the entire area in which the rigid body unit 320 can move with respect to the rotary shaft 210.

In addition, a total diameter of the first sealing unit 701 may be larger than a maximum area in which the rigid body unit 320 can move with respect to the rotary shaft 210.

Therefore, the rigid body unit 320 can move while always being supported by the first sealing body 711.

Specifically, a maximum radius of the first sealing body 711 may be larger than a radius obtained when the bottom surface of the rigid body unit 320 moves farthest from the outer circumferential surface of the first sealing body 711. The diameter of the first transmission hole 721 may be smaller than a diameter obtained when the bottom surface of the rigid body unit 320 moves closest to the inner circumferential surface of the sealing body 711.

Thus, the rigid body unit 320 can always shield the first transmission hole 721 and can be disposed inside the first sealing body 711.

Referring to FIG. 28(b), the rotary shaft 210 rotates so that the shaft of the bearing unit 310 is inclined upward, and the shaft of the rigid body unit 320 can also be arranged parallel to the shaft of the bearing unit 310.

The left part of the bottom surface of the rigid body unit 320 moves toward the outer circumferential surface of the first sealing body 711, and the right part of the bottom surface of the rigid body unit 320 moves toward the transmission hole 721 of the first sealing body 711.

Even at this time, the left part of the bottom surface of the rigid body unit 320 may be disposed farther inward than the outer circumferential surface of the first sealing body 711, and the right part of the lower surface of the rigid body unit 320 may be disposed farther outward than the first transmission hole 721.

Referring to FIG. 28(c), the rotary shaft 210 further rotates so that the shaft of the bearing unit 310 and the shaft of the rigid body unit 320 may be parallel to the rotary shaft 210 when viewed from the height direction.

Of course, even in this case, the outermost portion of the bottom surface of the rigid body unit 320 may be disposed farther inward than the outer circumferential surface of the first sealing body 711 with respect to the forward and backward directions, and the innermost portion of the rigid body unit 320 may be disposed farther outward than the first transmission hole 721.

Referring to FIG. 24(d), the rotary shaft 210 rotates so that the shaft of the bearing unit 310 is inclined downward, and the shaft of the rigid body unit 320 is also disposed parallel to the shaft of the bearing unit 310.

The left portion of the bottom surface of the rigid body unit 320 moves toward the transmission hole 721 of the first sealing body 711, and the right portion of the bottom surface of the rigid body unit 320 further moves toward the outer circumferential surface of the sealing body 711.

Even at this time, the right portion of the bottom surface of the rigid body unit 320 is disposed farther inward than the outer circumferential surface of the first sealing body 711, and the left portion of the bottom surface of the rigid body unit 320 is disposed farther outward than the first transmission hole 721.

As a result, the first sealing body 711 can support the rigid body unit 320 while shielding the passage hole 550, and the rigid body unit 320 can continuously shield the first transmission hole 721.

FIG. 29 is a view illustrating another example of the sealing unit 700 of the laundry treatment apparatus according to the present disclosure.

The sealing unit 700 may include a second sealing unit 702 coupled to the passage hole 550 and provided to contact the holder 400 or the power transmission unit 300.

Referring to FIG. 29(a), the second sealing unit 702 may include a sealing body 710 for shielding the passage hole 550 while being inserted into the holding plate 410, a passage hole 720 formed to penetrate the sealing body 710 so as to enable the holding plate 710 to pass therethrough, and a fixing unit 730 for coupling the sealing body 710 to the passage hole 550 or the support plate 510.

Referring to FIG. 29(b), the second sealing unit 702 may be provided to shield the plurality of passage holes 550 while being fitted into the plurality of holding plates 410.

The second sealing unit 702 may be entirely made of an elastic material so that the sealing body 710 can be deformed based on the passage hole 550.

Thus, the second sealing unit 702 can continuously seal the passage hole 550 while allowing the holding plate 410 to move back and forth.

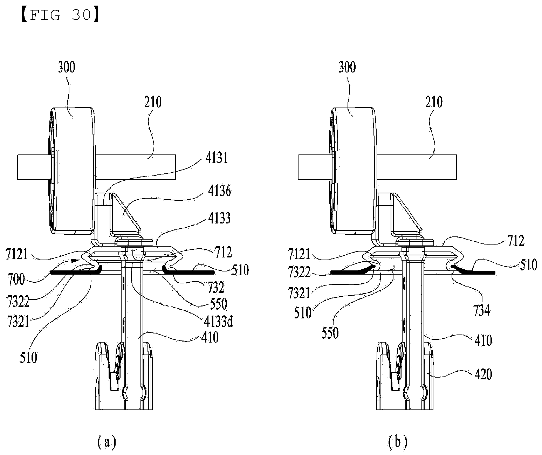

FIG. 30 is a view illustrating other examples of the second sealing unit 702 according to the present disclosure.

Commonly in all embodiments, the second sealing unit 702 may include a second sealing body 712 made of an elastic material.

The second sealing body 712 may be inserted into the holding plate 410 to seal the passage hole 550. For example, the second sealing body 712 may be provided to seal the passage hole 550 while being coupled to or seated on the support plate 510.

The second sealing body 712 may be provided as a corrugated pipe structure so as to be variable in shape or to be stretched or compressed in length.

In addition, the second sealing body 712 may have a larger area than the passage hole 550 to shield the passage hole 550, and may also be formed in a case shape capable of accommodating a portion of the holding plate 410.

The second sealing body 712 may have a corrugated pipe structure or a multi-stage structure extending upward from the passage hole 550.

The second transmission hole 722 may have the same cross-sectional shape as the holding plate 410, or may have a slightly smaller area than the holding plate 410. Accordingly, the second transmission hole 722 is forcibly fitted to the holding plate 410 to prevent hot air or steam from leaking outside the second transmission hole 722.

The second sealing unit 702 may include a second fixing unit 732 extending from the second sealing body 712 and coupled to the passage hole.

The second fixing unit 732 may include a contraction unit 7322 disposed below the second sealing body 712 to have a smaller diameter than the second sealing body 712, and an extension unit 7321 extending from a lower portion of the contraction unit 7322 to have a larger diameter than the contraction unit 7322.

Due to the contraction unit 7322 and the extension unit 7321, the second sealing unit 702 can be extended or contracted by a predetermined length in a width direction and/or a vertical direction like a corrugated pipe.

The cross-sectional area or width of the extension unit 7321 may be larger than the area or diameter of the passage hole 550, and the cross-sectional area or width of the second sealing body 712 may be greater than the area or diameter of the passage hole 550.

The second sealing body may be provided with a retaining ring 7121 which expands the area of the second sealing body 712 and maintains a much larger area than the contraction unit 7322.

The retaining ring 7121 may be integrally provided with the second sealing body 712, and may protrude from the inner circumferential surface of the second sealing body 712.

The top surface of the second sealing body 712 may be provided to support the holder support 4133. The inner circumferential surface of the second transmission hole 722 may be inserted into the recessed groove 4133a.

As a result, the top surface of the second sealing body 712 may be inserted into the recessed groove 4133a provided in the holding plate 410 such that the position of the second sealing body 712 can be fixed.

The holding plate 410 may include a support extension unit 4133d capable of supporting the top surface and the bottom surface of the second sealing body 712 in the recessed groove 4133a.

The support extension unit 4133d may have a larger diameter than the recessed groove 4133a.

Referring to FIG. 30(a), the extension unit 7321 may be seated on the outer circumferential surface of the passage hole 550 to shield the passage hole 550. The contraction unit 7322 may be disposed above the support plate 510.

This is because the holder 410 and the power transmission unit 400 are only pressed toward the support plate 510 by a load, but are not lifted upward. The total height of the second sealing body 712 may be greater in size than a spacing between the holder support 4133 and the support plate 510.

As a result, the second sealing unit 702 is seated on the support plate 510 where the passage hole 550 is disposed, seals the passage hole 550, and allows pendulum motion of the holder 400.

Referring to FIG. 30(b), the extension unit 7321 is disposed under the support plate 510, the contraction unit 7322 is disposed to be inserted into the passage hole 550, and the second sealing body 712 may be disposed above the passage hole 550.

As a result, the extension unit 7321 and the contraction unit 7322 may be inserted into the passage hole 550, and may be fitted into the passage hole 550.

Therefore, even if the holding plate 410 performs a pendulum motion or temporary vibration or impact is transmitted to the second sealing unit 702, the second sealing unit 702 can be prevented from being separated from the support plate 510.

It will be apparent to those skilled in the art that various modifications and variations can be made in the present disclosure without departing from the spirit or scope of the inventions. Thus, it is intended that the present disclosure covers the modifications and variations of this invention provided they come within the scope of the appended claims and their equivalents.

## Claims

1. A laundry treatment apparatus comprising:
a cabinet including an opening formed at a front side thereof;
an inner case provided in the cabinet to accommodate clothes therein;
a door rotatably coupled to the cabinet to open and close the opening;
a machine room provided below the inner case to generate hot air or steam to be supplied to an inside of the inner case;
a holder exposed to the inside of the inner case and allowing the clothes or a clothes hanger to be seated thereon;
a drive unit that includes a rotatory shaft extending in a width direction of the inner case to agitate the holder, and a motor configured to rotate the rotary shaft;
a power transmission unit coupled to the rotary shaft to support the holder; and
a support unit coupled to the cabinet or the inner case to support at least one of the rotary shaft, the power transmission unit, and the holder,
wherein the support unit is provided to be disposed lower than the rotary shaft.

2. The laundry treatment apparatus according to claim 1, wherein the support unit includes:
a support plate, both ends of which are fixed to the cabinet or the inner case and at least a portion of which is disposed lower than the rotary shaft.

3. The laundry treatment apparatus according to claim 2, wherein the support plate includes:
a support holder that supports at least one of the power transmission unit and the holder to distribute a load applied to the rotary shaft.

4. The laundry treatment apparatus according to claim 3, wherein the support holder includes:
a passage hole that passes through the support plate to allow any one of the power transmission unit and the holder to pass therethrough; and
a seating surface provided on an outer circumferential surface of the passage hole to support a bottom surface of the power transmission unit or the holder.

5. The laundry treatment apparatus according to claim 4, wherein the power transmission unit includes:
a bearing unit having an outer circumferential surface that is obliquely coupled to the rotary shaft so that an extension direction of the outer circumferential surface rotates about the rotary shaft; and
a rigid body coupled to the outer circumferential surface of the bearing unit to rotate independently of the bearing unit and configured to support the holder,
wherein the seating surface is provided as a curved surface so that the seating surface supports the rigid body unit while allowing movement of the rigid body unit.

6. The laundry treatment apparatus according to claim 5, wherein:
the seating surface is formed to be recessed from the support plate.

7. The laundry treatment apparatus according to claim 6, wherein:
a width or diameter of the seating surface is greater than a depth.

8. The laundry treatment apparatus according to claim 6, wherein:
a width of the seating surface is greater than a thickness of an outer circumferential surface of the rigid body unit.

9. The laundry treatment apparatus according to claim 6, wherein:
the seating surface is formed in a funnel shape.

10. The laundry treatment apparatus according to claim 6, wherein:
a radius of curvature of the seating surface is greater than or equal to a radius of curvature of a trajectory along which an outer circumferential surface of the rigid body unit moves.

11. The laundry treatment apparatus according to claim 6, wherein:
a radius of curvature of the seating surface is greater than or equal to a radius of curvature of an outer circumferential surface of the rigid body unit.

12. The laundry treatment apparatus according to claim 6, wherein the support holder includes:
a reinforcement unit protruding from the support plate toward the rigid body unit,
wherein the seating surface is provided inside the reinforcement unit.

13. The laundry treatment apparatus according to claim 12, wherein:
a height at which the reinforcement unit protrudes from the support plate is greater than a depth at which the seating surface is recessed.

14. The laundry treatment apparatus according to claim 4, wherein the power transmission unit includes:
a bearing unit having an outer circumferential surface that is obliquely coupled to the rotary shaft so that an extension direction of the outer circumferential surface rotates about the rotary shaft; and
a rigid body unit coupled to the outer circumferential surface of the bearing unit to rotate independently of the bearing unit and configured to support the holder,
wherein the seating surface is provided to support the holder.

15. The laundry treatment apparatus according to claim 14, wherein the holder includes:
a coupling plate coupled to the rigid body unit and formed to extend into the inner case by penetrating the passage hole,
wherein the coupling plate includes:
a coupling end coupled to the rigid body unit;
an extension end extending from the coupling end unit and exposed to the inside of the inner case by penetrating the passage hole;
a holder support unit protruding to be larger than a diameter of the passage hole and seated on the seating surface.

16. The laundry treatment apparatus according to claim 15, wherein the holder support includes:
a holder body provided to have a larger thickness than the coupling end unit; and
a body surface provided at a lower portion of the holder body and supported by the seating surface.

17. The laundry treatment apparatus according to claim 16, wherein:
the seating surface is provided to be larger than a region of the body surface; and
a radius of curvature of the seating surface is equal to or greater than a radius of curvature of the body surface.

18. The laundry treatment apparatus according to claim 2, wherein the support unit includes:
a support pillar coupled to the support plate and supporting a lower portion of the rotary shaft.

19. The laundry treatment apparatus according to claim 18, wherein the support pillar includes:
a pillar body coupled to the support plate; and
a pillar groove provided at an upper portion of the pillar body to receive and support the rotary shaft.

20. The laundry treatment apparatus according to claim 18, wherein:
the support pillar is implemented as a plurality of support pillars that is spaced apart from each other by a preset distance in a longitudinal direction of the rotary shaft.

21. The laundry treatment apparatus according to claim 18, further comprising:
a power transmission unit that is coupled to the rotary shaft to fix the holder and transmits the power to the holder,
wherein the support plate further includes:
a passage hole through which one of the power transmission unit and the holder pass and supporting the one of the power transmission unit and the holder,
the plurality of support pillars is arranged to be spaced apart from the passage hole.

22. The laundry treatment apparatus according to claim 2, wherein the support unit further includes:
a rotary coupling unit coupled to the support plate to rotatably support both ends of the rotary shaft.

23. A laundry treatment apparatus comprising:
a cabinet including an opening formed at a front side thereof;
an inner case provided in the cabinet to accommodate clothes therein;
a door rotatably coupled to the cabinet to open and close the opening;
a machine room provided below the inner case to generate hot air or steam to be supplied to an inside of the inner case;
a holder exposed to the inside of the inner case and allowing the clothes or a clothes hanger to be seated thereon;
a drive unit including a rotary shaft that supports a load of the holder and provided to agitate (wobble or sway) the holder, and a motor that provides the rotary shaft with power for shaking the holder; and
a support unit supported by the cabinet or the inner case to distribute or support a load applied to the rotary shaft,
wherein the support unit is provided to contact the rotary shaft at a lower position than the rotary shaft or to accommodate and support the rotary shaft.

24. The laundry treatment apparatus according to claim 23, wherein the support unit includes:
a support plate provided below the rotary shaft; and
a support pillar provided on the support plate and supporting a lower portion of the rotary shaft.

25. The laundry treatment apparatus according to claim 24, wherein:
the support pillar is provided to support a region between both ends of the rotary shaft.

26. The laundry treatment apparatus according to claim 24, wherein:
the support plate includes a rotary coupling unit configured to rotatably support both ends of the rotary shaft; and
the support pillar is disposed between the rotary coupling units.

27. The laundry treatment apparatus according to claim 24, wherein:
the support plate includes a through-hole through which the holder passes;
the support pillar is arranged to be spaced apart from the through-hole.

28. A laundry treatment apparatus comprising:
a cabinet including an opening formed at a front side thereof;
an inner case provided in the cabinet to accommodate clothes therein;
a door rotatably coupled to the cabinet to open and close the opening;
a machine room provided below the inner case to generate hot air or steam to be supplied to an inside of the inner case;
a holder exposed to the inside of the inner case and allowing the clothes or a clothes hanger to be seated thereon;
a drive unit that includes a rotatory shaft extending in a width direction of the inner case to agitate the holder, and a motor configured to rotate the rotary shaft;
a power transmission unit coupled to the rotary shaft to support the holder; and
a support unit provided to support any one of the power transmission unit and the holder to distribute a load applied to the rotary shaft.

29. The laundry treatment apparatus according to claim 28, wherein the support unit includes:
a support plate fixed to the cabinet and disposed at a lower position than the rotary shaft;
a passage hole formed to penetrate the support plate to allow any one of the power transmission unit and the holder to pass therethrough; and
a support holder extending from an outer circumferential surface of the through-hole to support at least a portion of the power transmission unit and the holder disposed above the through hole.
